# EUROPEAN PATENT SPECIFICATION

(11) **EP 2 397 186 B1**
(45) Date of publication and mention of the grant of the patent: **24.01.2018**
(21) Application number: 11075176.5
(22) Date of filing: 12.12.2005
(51) Int. Cl.: A61N 1/37, A61B 5/0452, A61B 5/00, A61N 1/368

(54) **Cardiac capture verification**
Verifizierung der Erfassung eines Herzsignals
Vérification de capture cardiaque

(30) Priority: 15.12.2004 US 12430; 15.12.2004 US 12433; 15.12.2004 US 12443; 15.12.2004 US 12608; 15.12.2004 US 12692
(43) Date of publication of application: 21.12.2011
(62) Divisional of application: 05853683.0
(73) Proprietor: Cardiac Pacemakers, Inc., St. Paul MN 55112-5798 (US)
(72) Inventor: Garg, Ankur, Minneapolis, Minnesota 55403 (US); Daum, Douglas R., Woodbury, Minnesota 55129 (US); Zhang, Geng, Newbury Park, California 91320 (US)
(74) Representative: Pfenning, Meinig & Partner mbB

(56) References cited:
- US-A1- 2002 120 206
- US-A1- 2002 183 637
- US-A1- 2003 050 671
- US-A1- 2004 082 975
- US-A1- 2004 243 014
- US-B1- 6 456 881

## Description

### FIELD OF THE INVENTION

The present invention relates generally to implantable medical devices and, more particularly, to cardiac rhythm management devices providing cardiac capture verification and management of retrograde signals. The present invention in particular concerns a medical system for classifying a cardiac response to a pacing pulse.

### BACKGROUND OF THE INVENTION

When functioning normally, the heart produces rhythmic contractions and is capable of efficiently pumping blood throughout the body. However, due to disease or injury, the heart rhythm may become irregular resulting in diminished pumping efficiency. Arrhythmia is a general term used to describe heart rhythm irregularities arising from a variety of physical conditions and disease processes. Cardiac rhythm management systems, such as implantable pacemakers and cardiac defibrillators, have been used as an effective treatment for patients with serious arrhythmias. These systems typically include circuitry to sense electrical signals from the heart and a pulse generator for delivering electrical stimulation pulses to the heart. Leads extending into the patient's heart are connected to electrodes that couple to the myocardium for sensing the heart's electrical signals and for delivering stimulation pulses to the heart in accordance with various therapies.

Cardiac rhythm management systems may operate to stimulate the heart tissue adjacent to the electrodes to produce a contraction of the tissue. Pacemakers are cardiac rhythm management systems that deliver a series of low energy pace pulses timed to assist the heart in producing a contractile rhythm that maintains cardiac pumping efficiency. Pace pulses may be intermittent or continuous, depending on the needs of the patient. There exist a number of categories of pacemaker devices, with various modes for sensing and pacing one or more heart chambers.

When a pace pulse produces a contraction in the heart tissue, the electrical cardiac signal following the contraction is denoted the captured response. The captured response may include an electrical signal, denoted the evoked response signal, associated with the heart contraction, along with a superimposed signal associated with residual post pace polarization at the electrode-tissue interface. The magnitude of the residual post pace polarization signal, or pacing artifact, may be affected by a variety of factors including lead polarization, after-potential from the pace pulse, lead impedance, patient impedance, pace pulse width, and pace pulse amplitude, for example.

A pace pulse must exceed a minimum energy value, or capture threshold, to produce a contraction. It is desirable for a pace pulse to have sufficient energy to stimulate capture of the heart without expending energy significantly in excess of the capture threshold. Thus, accurate determination of the capture threshold may be required for efficient pace energy management. If the pace pulse energy is too low, the pace pulses may not reliably produce a contractile response in the heart and may result in ineffective pacing. If the pace pulse energy is too high, the patient may experience discomfort and the battery life of the device will be shorter.

Capture detection allows the cardiac rhythm management system to adjust the energy level of pace pulses to correspond to the optimum energy expenditure that reliably produces a contraction. Further, capture detection allows the cardiac rhythm management system to initiate a back-up pulse at a higher energy level whenever a pace pulse does not produce a contraction.

In some situations, pacing pulses delivered to the heart may cause various undesirable phenomena which inhibit capture and/or confuse capture detection processes. As one example, effective pacing may be compromised due to retrograde signals initiated by a pacing pulse. For example, when a device performs atrial sensing, a retrograde P-wave be sensed when a depolarization wave initiated in a ventricle by a pacing pulse or intrinsic activation travels back to the atrium. Retrograde P-waves may inhibit atrial pacing. A pacing pulse delivered to the atrium will not result in capture if the atrial tissue is refractory due to a retrograde P-wave. Further, in some scenarios, retrograde conduction to the atrium may cause pacemaker mediated tachycardia.

At times, a pacing pulse may merge with an intrinsic beat, producing a fusion beat. A fusion beat is a cardiac contraction that occurs when two cardiac depolarizations of a particular chamber, but from separate initiation sites, merge. When the heart is being paced, a fusion beat may occur when an intrinsic cardiac depolarization of a particular chamber merges with a pacemaker output pulse within that chamber. Fusion beats, as seen on electrocardiographic recordings, exhibit various morphologies. The merging depolarizations of a fusion beat do not contribute evenly to the total depolarization.

Pseudofusion occurs when a pacemaker output pulse is superimposed upon a spontaneous P wave during atrial pacing or upon a spontaneous QRS complex during ventricular pacing. In pseudofusion, the pacing stimulus may be ineffective because the tissue around the electrode has already spontaneously depolarized and is in its refractory period. Fusion or pseudofusion beats may cause false detection of capture and may lead to erroneous capture threshold values.

US 2004/0243014 describes methods and systems for creating, adjusting and using cardiac waveform morphology templates. Target regions associated with a cardiac waveform used to establish a template representing a particular beat morphology may be established based on an average of the feature coordinates for each cardiac waveform. US 200310050671 describes an apparatus and method for identifying fusion beats as part of an automatic capture routine in a cardiac stimulation device. Fusion identification is performed using both template matching and assessment of particular detection features. US 2004/0082975 describes an implantable medical device verifying capture in multiple chambers of the heart. A template characterizing an evoked response may be used to verify capture in individual heart chambers or multiple heart chambers. US 2002/0183637 describes an implantable medical device generating a template representative of one beat of a patient's normal cardiac rhythm. Template generation methodology typically involves averaging or median filtering aligned shock channels. US 2002/0120206 describes methods of distinguishing between different types of cardiac abnormalities. Sample values within a time window of respective cardiac cycles may be averaged or medianized to form a template using an identified R-wave as a reference point.

### SUMMARY OF THE INVENTION

The present invention provides a medical system according to the independent Preferred embodiments are defined by the dependent claims.

Aspects, embodiments, implementations or examples of the present disclosure which do not fall under the scope of the appended claims do not form part of the present invention. The present disclosure describes a cardiac medical system configured to classify cardiac responses to pacing pulses using retrograde analysis. The system includes a plurality of implantable electrodes that may be used for sensing a cardiac signal and/or delivering cardiac pacing pulses to a patient. Controller circuitry coupled to the electrodes is disposed within an implantable housing. The controller is configured to deliver a pacing pulse to an atrium of the patient's heart during a cardiac cycle. The controller senses for an intrinsic activation of a ventricle during the cardiac cycle and delivers a pacing pulse to the ventricle if the intrinsic activation is not sensed. The controller senses in the atrium for a retrograde P-wave in response to the ventricular pacing pulse or the intrinsic activation. The controller classifies the cardiac response to the atrial pacing pulse as a non-captured response if the retrograde P-wave is detected.

According to one implementation, if the controller does not detect the retrograde P-wave, the controller classifies the cardiac response to the atrial pacing pulse as a possible captured response. The controller may be further configured to sense for an atrial evoked response associated with the atrial pacing pulse. According to one implementation, the controller classifies the cardiac response as a captured response if the atrial evoked response is detected. In another implementation, the controller classifies the cardiac response to the pacing pulse as a non-captured response if the atrial evoked response is not detected and the retrograde P-wave is detected. In yet a further implementation, the controller classifies the cardiac response to the pacing pulse as a captured response if the atrial evoked response is detected and the retrograde P-wave is not detected.

The controller may sense for the retrograde P-wave during a time interval following sensing the intrinsic ventricular activity or delivery of the ventricular pacing pulse, such as during a post ventricular atrial refractory period.

Additional atrial pacing pulses may be delivered by the controller during cardiac cycles subsequent to the cardiac cycle, with additional ventricular pacing pulses delivered and/or intrinsic ventricular activations sensed during these additional cycles. The controller senses in the atrium for retrograde P-waves responsive to the additional ventricular pacing pulses or the intrinsic ventricular activations, and classifies cardiac responses to the additional atrial pacing pulses as non-captured responses if the retrograde P-waves are detected. The controller may be configured to classify the cardiac responses to the atrial pacing pulses beat by beat during a pacing therapy delivered to the patient or during a capture threshold test, for example.

Also described is a cardiac medical system that includes a plurality of implantable electrodes configured to electrically couple to the patient's heart. The electrodes are used to sense cardiac signals and/or deliver cardiac pacing pulses. A controller, disposed in an implantable housing, is coupled to the electrodes. The controller is configured to deliver a pacing pulse to an atrium of the patient's heart during a cardiac cycle and determine if the atrial pacing pulse captured the atrium. The controller senses for an intrinsic activation of a ventricle during the cardiac cycle and delivers a pacing pulse to the ventricle if the intrinsic ventricular activation is not detected. The controller delivers another pacing pulse timed to reduce atrial retrograde conduction if the atrial pacing pulse did not capture the atrium, wherein the atrial retrograde conduction is responsive to the ventricular pacing pulse or the intrinsic ventricular activation.

In one implementation, the controller senses for an atrial evoked response and classifies the cardiac pacing response as a non-captured response if the atrial evoked response is not detected. The pacing pulse timed to reduce atrial retrograde conduction may comprise a backup pace delivered to the atrium.

In another implementation, the controller senses for a retrograde P-wave and classifies the cardiac pacing response as a non-captured response if the retrograde P-wave is detected.

In yet another implementation, the pacing pulse timed to reduce atrial retrograde conduction comprises a delayed atrial pacing pulse of a subsequent cardiac cycle. The controller is configured to delay a scheduled atrial pacing pulse for the subsequent cardiac cycle to reduce retrograde conduction during the subsequent cardiac cycle if the retrograde P-wave is detected and if the scheduled atrial pacing pulse is scheduled to occur before expiration of an atrial effective refractory period. The delayed atrial pacing pulse may be delivered with increased energy beyond a previously scheduled energy. The scheduled atrial pacing pulse may be delivered as scheduled if the scheduled atrial pacing pulse is not scheduled to occur before expiration of an atrial effective refractory period. The atrial effective refractory period may be modified by the controller if the delayed atrial pacing pulse does not produce capture.

Also described is a medical system including a pacing circuit configured to deliver pacing pulses to a heart chamber and a sensing circuit configured to sense cardiac signals associated with the pacing pulses. The medical system also includes a processor coupled to the sensing circuit. The processor is configured to determine a cardiac response classification interval based on a timing variability of a feature of cardiac signals associated with a type of cardiac pacing response. The cardiac response classification interval defines a period of time after a pacing pulse that a cardiac signal is sensed to determine a response to a pacing pulse. For example, the feature associated with a type of cardiac pacing response may involve a peak amplitude and the response to the pacing pulse may comprise a captured response

According to some configurations, the processor determines the standard deviation of the feature timing. Using one approach, the processor forms the cardiac response classification interval based on the average feature timing and the standard deviation. The processor may determine values for the timing of the feature for each of the cardiac signals and average the feature timing values. The processor may be arranged to adapt the cardiac response classification interval until a stability criterion is achieved. For example, the processor may adapt one or both of an upper bound and a lower bound of the cardiac response classification interval until predetermined stability criteria are achieved. According to one aspect, a cardiac response classification processor is configured to use the cardiac response classification interval to determine a cardiac response to a
pacing pulse, such as discriminating between at least two of capture, non-capture, and fusion using the cardiac response classification interval.

According to another embodiment, a medical system includes a pacing circuit configured to deliver pacing pulses to a heart chamber and a sensing circuit configured to sense cardiac signals associated with the pacing pulses. The medical system also includes a template processor coupled to the sensing circuit. The template processor determines a value associated with a cardiac signal feature for each sensed cardiac signal, determines a median value of the cardiac signal feature values, and generates a cardiac response template based on the median value.

For example, the processor may determine a peak amplitude for each sensed cardiac signal and determine a median value of the peak amplitudes of the sensed cardiac signals. The median value of the peak amplitudes may be used to generate the cardiac response template. In another example, the value comprises a timing of a peak amplitude of each sensed cardiac signal. The processor may use the cardiac response template to detect capture or other cardiac responses to pacing.

According to one aspect, the pacing circuit delivers pacing pulses above a capture threshold. The sensing circuit senses the cardiac signals associated with the pacing pulses delivered above the capture threshold. The template processor generates a captured response template. According to another aspect, the pacing circuit delivers the pacing pulses below a capture threshold. The sensing circuit senses cardiac signals associated with the pacing pulses delivered below the capture threshold. The template processor generates a non-captured response template.

In some configurations, the pacing rate of the pacing pulses is jittered and/or is delivered at a rate exceeding an intrinsic rate of the heart chamber. The pacing circuit may be configured to deliver a backup pace following each pacing pulse.

In some configurations, the sensing circuit is further configured to sense each cardiac signal in a time interval prior to delivery of a pacing pulse. The template processor forms a baseline using the cardiac signal sensed in the time interval prior to the delivery of the pacing pulse. The baseline is used to determine the value associated with the cardiac signal feature.

Also described is a medical system including a pacing circuit configured to deliver a cardiac pacing pulse to a patient and a sensing circuit configured to a sense cardiac signal associated with the pacing pulse. A cardiac response processor is coupled to the sensing circuit. The cardiac response processor establishes a baseline amplitude of the cardiac signal using data acquired in a time interval prior to delivery of the pacing pulse and determines a cardiac response to the pacing pulse using the baseline amplitude of the cardiac signal. The pacing pulse may be an atrial or a ventricular pacing pulse.

The cardiac signal may be filtered prior to establishing the baseline amplitude. In one example, cardiac signal data is averaged within the time interval.

According to some aspects, the time interval may be a moving time interval having a range of about 3 msec to about 10 msec.

According to some aspects, the cardiac response processor is configured to establish the baseline amplitude for each paced beat.

The cardiac response processor may measure a peak amplitude of the cardiac signal referenced from the baseline amplitude and use the peak amplitude referenced from the baseline amplitude to determine the cardiac response to the pacing pulse.

Also described is a medical system used to determine cardiac responses to pacing. The system includes a pacing circuit configured to deliver pacing pulses to a heart chamber. A sensing circuit is configured to sense a cardiac signal associated with a pacing pulse. A processor coupled to the sensing circuit is configured to detect a feature of the cardiac signal and determine a variability of the feature with respect to one or more previously detected cardiac signal features. The processor classifies the cardiac response based on the feature variability.

The above summary of the present invention is not intended to describe each embodiment or every implementation of the present invention. Advantages and attainments, together with a more complete understanding of the invention, will become apparent and appreciated by referring to the following detailed description and claims in conjunction with the accompanying drawings.

### BRIEF DESCRIPTION OF THE DRAWINGS

Figure 1A is a graph illustrating a method of confirming atrial loss of capture;
Figure 1B is a flowchart illustrating a method of atrial capture verification;
Figure 1C is a flowchart illustrating a method of determining a cardiac response to a pacing pulse using both an atrial evoked response and retrograde P-wave detection;
Figure 1D is a flowchart illustrating a method of retrograde management;
Figure 1E is a flowchart illustrating in more detail a retrograde conduction management method;
Figures 1F and 1G illustrate the operation of a retrograde management method;
Figure 2 illustrates an implantable cardiac rhythm management system that may be used in connection with atrial capture verification methodologies;
Figure 3 is a block diagram of an implantable medical device that may be used to verify atrial capture and manage atrial retrograde conduction;
Figure 4A is a flowchart of a method of capture detection based on retrograde conduction;
Figure 4B is a flowchart of a method of retrograde management;
Figure 4C is a flowchart of a method of capture detection and retrograde management;
Figure 5A is a flowchart illustrating a method of forming a cardiac response classification interval;
Figure 5B is a graph illustrating a classification response interval oriented with respect to a feature of a captured response signal;
Figure 6A illustrates an initialized cardiac response classification interval;
Figures 6B and 6C illustrate a cardiac response classification interval before and after adaptation, respectively;
Figure 7 is a flowchart illustrating a method of classifying a cardiac response to pacing using an adaptable classification interval;
Figure 8 is a flowchart illustrating a method of stabilizing a cardiac response classification interval;
Figure 9 is a flowchart illustrating a method of discriminating between capture and fusion based on the variability of a cardiac signal feature;
Figure 10 is a flowchart illustrating a method of performing a capture threshold test with optional fusion management or hysteresis search;
Figure 11 is a flowchart illustrating a method of beat-to-beat automatic capture verification with optional fusion management or hysteresis search;
Figure 12 is a graph illustrating cardiac signal features that may be utilized for discriminating between capture and fusion;
Figure 13 is a block diagram of an implantable medical device that may be used to initiate and adapt a cardiac response classification interval and classify a cardiac response to pacing;
Figure 14 is a graph illustrating two consecutive heartbeats including a captured response and a non-captured response;
Figure 15 is a flow chart of a method of initializing a capture template;
Figure 16 is a flow chart of a method of initializing a non-capture template;
Figure 17 is a flow chart of a method of adapting a baseline; and
Figure 18 is a block diagram of an implantable medical device that may be used to initialize cardiac pacing response detection and/or adapt baselines for cardiac pacing response detection.

While the invention is amenable to various modifications and alternative forms, specifics thereof have been shown by way of example in the drawings and will be described in detail below. It is to be understood, however, that the intention is not to limit the invention to the particular embodiments described. On the contrary, the invention is intended to cover all modifications, equivalents, and alternatives falling within the scope of the invention as defined by the appended claims.

### DETAILED DESCRIPTION OF VARIOUS EMBODIMENTS

After delivery of a pacing pulse to a heart chamber, various cardiac responses to the pacing pulse are possible. In one scenario, the pacing pulse may generate a propagating wavefront of depolarization resulting in a contraction of the heart chamber. In this scenario, the pacing pulse is said to have captured the heart chamber. Capture of the heart chamber may occur if the pacing pulse has sufficient energy and is delivered during a non-refractory period. If the pacing pulse does not produce contraction of the chamber, the cardiac response is referred to as non-capture or loss of capture. Non-capture may occur, for example, if the pacing pulse energy is too low, and/or if the pacing pulse is delivered during a refractory period of the cardiac tissue.

By way of example, the processes of the present disclosure may be used in capture threshold testing to determine an energy used for pacing. The minimum pacing energy that produces capture is referred to as the capture threshold. It is desirable for a pace pulse to have sufficient energy to capture the heart without expending excess energy above the capture threshold. Thus, accurate determination of the capture threshold may be desirable for efficient pacing.

Those skilled in the art will appreciate that reference to a capture threshold testing procedure indicates a method of determining the capture threshold in one or more of the left atrium, right atrium, left ventricle, and right ventricle. In such a procedure, the pacemaker, automatically or upon command, initiates a search for the capture threshold of the selected heart chamber. The capture threshold is defined as the lowest pacing energy that consistently captures the heart.

In one example of an automatic capture threshold procedure, the pacemaker delivers a sequence of pacing pulses to the heart and detects the cardiac responses to the pace pulses. The energy of the pacing pulses may be decreased in discrete steps until a predetermined number of loss-of capture responses occur. The pacemaker may increase the stimulation energy in discrete steps until a predetermined number of capture responses occur to confirm the capture threshold. A capture threshold test may be performed using cardiac response classification methods of the present invention.

Other procedures for implementing capture threshold testing may be utilized. In one example, the pacing energy may be increased in discrete steps until capture is detected. In another example, the pacing energy may be adjusted according to a binomial search pattern.

Capture threshold determination is distinguishable from automatic capture detection, a procedure that typically occurs on a beat-by-beat basis during pacing. Automatic capture detection verifies that a delivered pace pulse results in a captured response. When a captured response is not detected following a pace pulse, the pacemaker may deliver a back up safety pace to ensure consistent pacing. The back up pace may be delivered, for example, about 70-80 ms after the initial pace pulse. If a predetermined number of pace pulses delivered during normal pacing do not produce a captured response, the pacemaker may initiate a capture threshold test to determine the capture threshold. Alternatively, if a predetermined number of pacing pulses do not produce a captured response, the pacemaker may adjust the pacing energy for the next pacing pulse. Atrial capture verification and atrial retrograde management may be implemented using processes of the present invention. The present disclosure is directed to methods and systems for determining if an atrial pacing pulse captures or fails to capture the atrium. Loss of capture determination may be based on detection of a ventricular depolarization wave that travels back to the atrium, denoted retrograde conduction. This retrograde depolarization wave may be sensed and used in capture verification processes of the present invention.

When a ventricular depolarization occurs, either intrinsically, or as a result of a ventricular pace, the depolarization wavefront may travel towards the atrium if the atrial tissue is not in its refractory period. If the myocardial tissue of the atrium is not refractory (i.e., if there was no intrinsic P-wave or the atrium was not captured by an atrial pacing pulse preceding the ventricular depolarization), then the wavefront initiated by the ventricular depolarization is more likely to be retrogradely conducted and sensed in the atrium as a retrograde P-wave. Thus, sensing a retrograde P-wave indicates that the atrial pacing pulse did not capture the atrium.

Further embodiments involve approaches for avoiding or managing atrial retrograde conduction when an atrial pacing pulse does not capture the atrium. According to some embodiments, a back up pace can be delivered to the atrium following loss of capture event. Backup pacing may be delivered at a relatively high energy level to ensure capture and thus prevent retrograde conduction to the atrium.

Other retrograde management approaches described herein involve delaying the next scheduled pace if a retrograde P-wave is detected. Following detection of the retrograde P-wave, the next scheduled atrial pace may be delayed until expiration of an effective atrial refractory period. Delaying the next scheduled pace allows the myocardium to recover from its refractory period caused by to the retrograde conduction before the next pacing pulse is delivered.

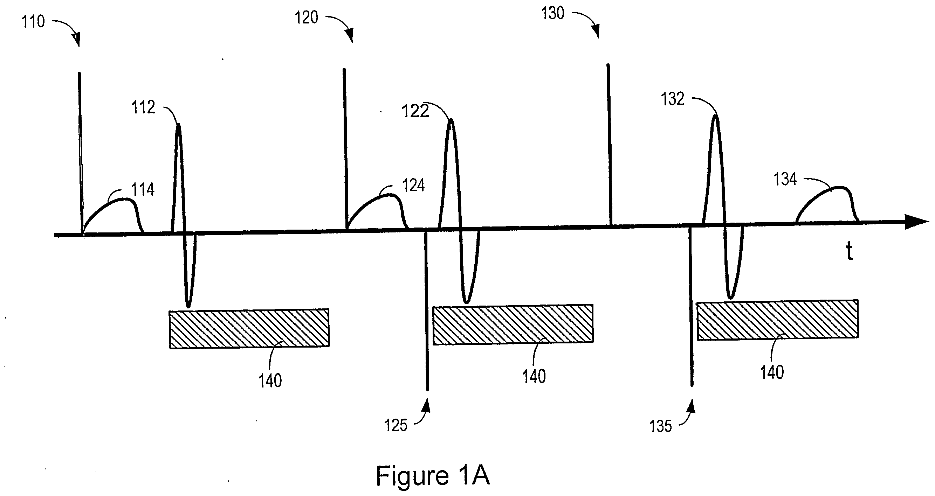

Figure 1A is a graph illustrating a method of confirming atrial loss of capture. This method may be used, for example, in a dual chamber device. When atrial capture is lost, AV synchrony may be disrupted, and a ventricular pulse may occur after the failed atrial pulse when an atrioventricular delay expires. Because the ventricle is activated before the atrium, the excitation may travel up to activate the atrium as retrograde conduction.

In the graph of Figure 1A, an atrial pacing pulse 110 that captures the atrium is followed by an evoked atrial response (AER) 114, and an intrinsic ventricular response 112, which is a QRS complex. A post ventricular atrial refractory period (PVARP) 140 is illustrated following the intrinsic ventricular response 112. This is an atrial paced heartbeat with intrinsic ventricular response.

The next heartbeat begins with an atrial pacing pulse 120 that captures the atrium. The atrial pacing pulse 120 is followed by an atrial evoked response 124. A ventricular pacing pulse 125 is delivered which captures the ventricle producing a ventricular evoked response 122. The ventricular evoked response shows a widened QRS complex relative to the intrinsic ventricular response 112. A PVARP 140 is illustrated following the ventricular pacing pulse 125.

The final heartbeat of Figure 1A illustrates an atrial retrograde conduction. The atrial pacing pulse 130 does not capture the atrium, thus an atrial evoked response is not sensed. The atrial pacing pulse 130 is followed, after an atrioventricular delay, by a ventricular pacing pulse 135, and a ventricular evoked response 132, showing a widened QRS complex relative to the intrinsic ventricular response 112. A PVARP 140 is illustrated following the ventricular pulse 135. An atrial retrograde P-wave 134 is illustrated within the PVARP 140 following the ventricular pulse 135. The retrograde P-wave 134 is produced by a depolarization wavefront initiated by the ventricular pulse 135 and conducted to the atrium. If retrograde conduction occurs in close proximity to a scheduled atrial pacing pulse, capture of the atrium may not occur even if the pulse energy is greater than a capture threshold.

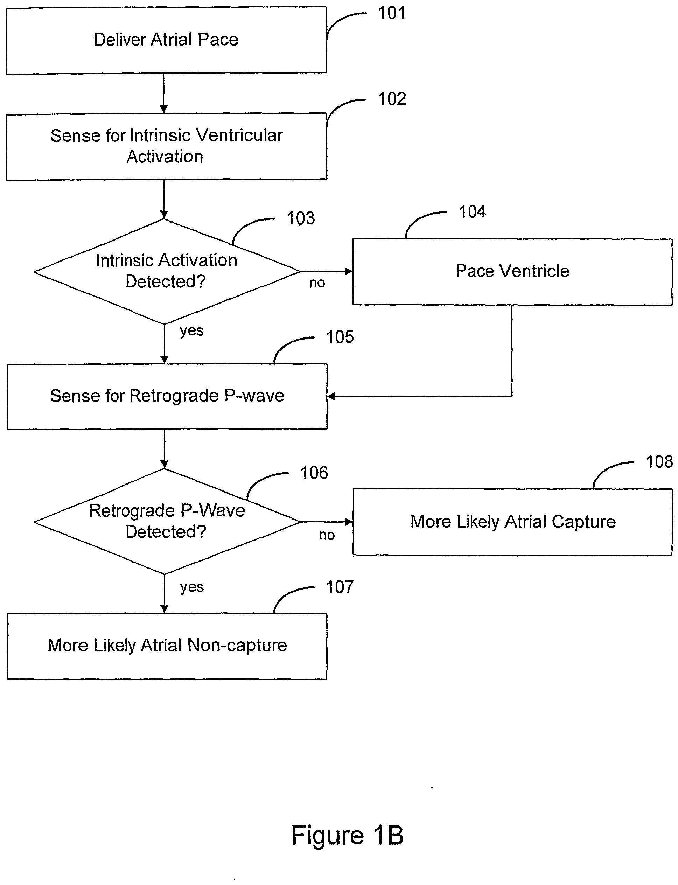

The atrial evoked response and/or retrograde P-wave occurring during a cardiac cycle may be utilized both for atrial capture verification and/or atrial retrograde management. Figures 1B-1C are flowcharts illustrating atrial capture verification. The flowchart of Figure 1B illustrates capture verification based on a sensed retrograde P-wave. A pacing pulse is delivered 101 to an atrium during a cardiac cycle. The system senses for 102 an intrinsic activation of a ventricle during the cardiac cycle. If intrinsic ventricular activation is not sensed 103, then a pacing pulse is delivered 104 to the ventricle.

The system senses for 105 a retrograde P-wave responsive to the intrinsic ventricular activation or the ventricular pace. If the retrograde P-wave is detected 106, then atrial non-capture is more likely 107. If the retrograde P-wave is not detected 106, then atrial capture is more likely, and the system may classify 108 the cardiac response to the atrial pace as a captured atrial response.

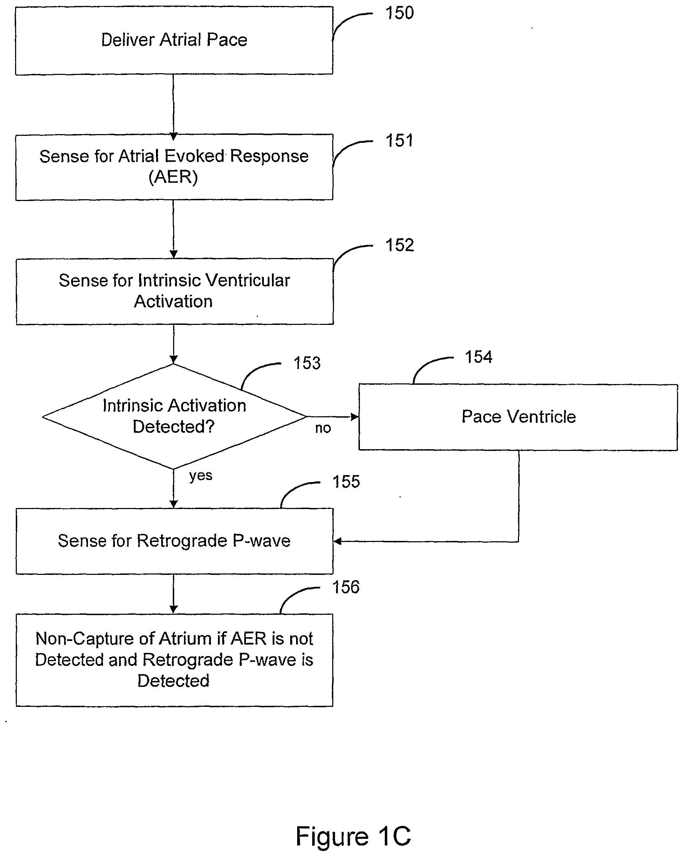

The flowchart of Figure 1C illustrates a method of determining a cardiac response to a pacing pulse using both an atrial evoked response and retrograde P-wave detection. Use of both the atrial evoked response and retrograde P-wave detection enhances the accuracy of capture verification processes. An atrial pacing pulse is delivered 150. The system senses for 151 an atrial evoked response following the atrial pacing. The system senses for 152 an intrinsic ventricular depolarization. A pacing pulse is delivered 154 to the ventricle if an intrinsic activation of the ventricle is not detected 153.

Following delivery of the ventricular pace or the intrinsic ventricular depolarization, the system senses in the atrium for 155 a retrograde P-wave. The cardiac response to the atrial pacing pulse is classified 156 as a non-captured response if the retrograde P-wave is detected and the atrial evoked response is not detected.

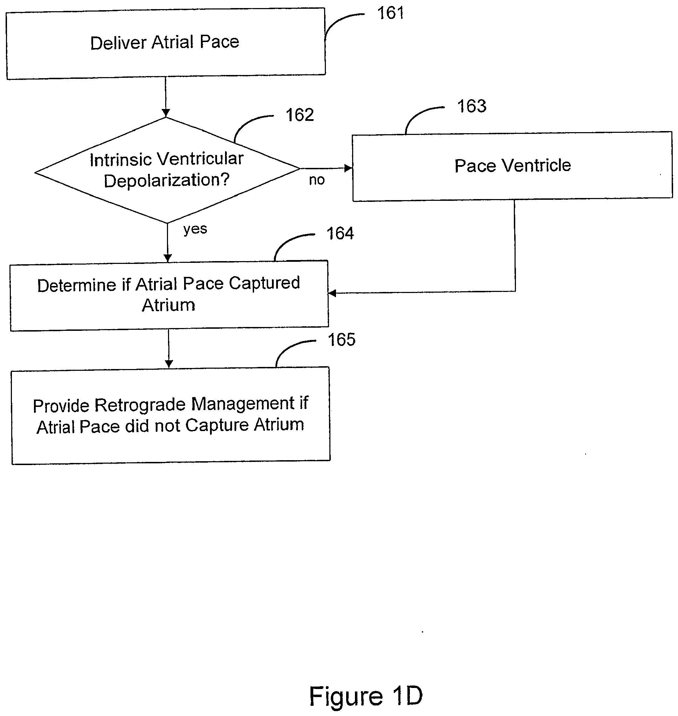

As previously discussed, non-capture of the atrium may result in a series of retrograde P-waves which are undesirable for effective pacing. Various embodiments involve the providing retrograde management if atrial non-capture is detected. The flowchart of Figure 1D illustrates a method of retrograde management in accordance with embodiments of the invention. A pacing pulse is delivered 161 to an atrium during a cardiac cycle. If intrinsic activation of the ventricle is not sensed 162 during the cardiac cycle, then a pacing pulse is delivered 163 to the ventricle. The depolarization wavefront initiated by the intrinsic ventricular activation or the ventricular pacing pulse may cause retrograde conduction to the atrium if the atrial pacing pulse did not capture the atrium. The system determines if 164 the atrial pacing pulse captured the atrium. If the system determines that the pacing pulse did not capture the atrium, then atrial retrograde management is provided 165.

The system may detect non-capture if an atrial evoked response is not detected. In this implementation, providing atrial retrograde management may involve delivering an atrial backup pace delivered shortly after the primary atrial pacing pulse. The system may determine that the atrial pacing pulse did not capture the atrium based on a detected retrograde P-wave. In this implementation, providing retrograde management may involve delaying a next scheduled atrial pacing pulse until after expiration of an atrial effective refractory period as described in more detail herein.

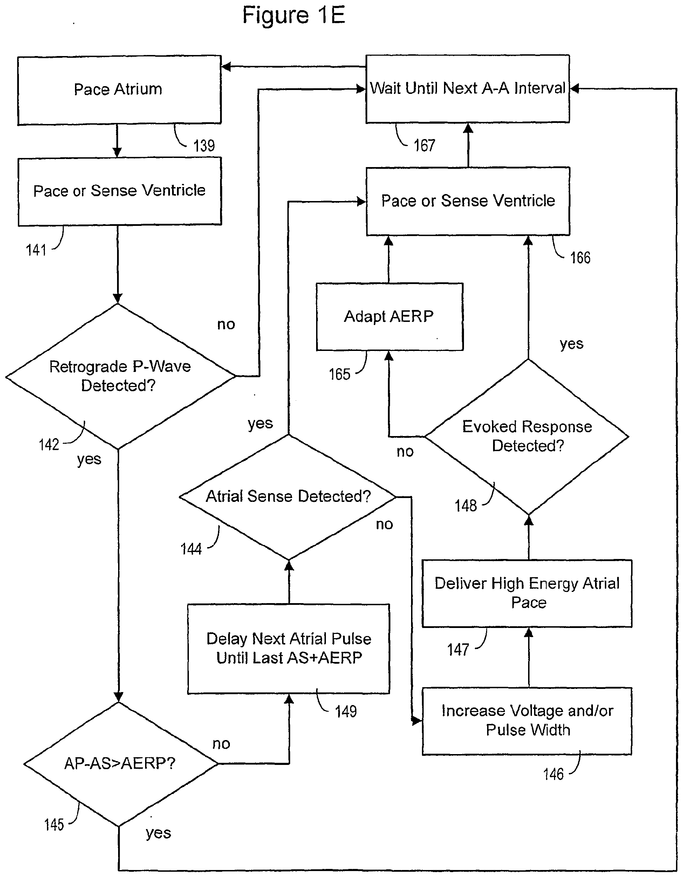

Figure 1E is a flowchart illustrating in more detail a retrograde conduction management method. In this example, the system senses for a retrograde P-wave and determines that an atrial pacing pulse did not produce capture of the atrium if the retrograde P-wave is sensed. The retrograde P-wave may cause the next scheduled atrial pace to be delivered while the atrial tissue is refractory. The scheduled atrial pace will be ineffective because the atrial tissue will not be able to react to the scheduled atrial pace. The system avoids delivery of atrial pacing while the atrial tissue is refractory by delaying delivery of the next scheduled atrial pace until after expiration of an atrial effective refractory period (AERP).

In the flowchart of Figure 1E, the system delivers 139 a next scheduled atrial pacing pulse and delivers a ventricular pace if an intrinsic ventricular depolarization is not sensed 141 during an AV interval. Following delivery of the ventricular pace or intrinsic ventricular depolarization, for example, during PVARP, the system senses for a retrograde P-wave. If a retrograde P-wave is not detected 142, then retrograde management is not necessary for the next cycle and the system delivers 139 the next scheduled atrial pulse after an A-A interval has expired 167.

If the retrograde P-wave is detected 142, a check is performed to determine if the time between the retrograde P-wave and the next scheduled atrial pace is longer than an atrial effective refractory period (AERP). For example, a suitable AERP may vary from patient to patient and may be about 200 to about 300 milliseconds. If the time is shorter than the AERP, the next atrial pacing pulse is rescheduled 149 by delaying it to occur after the AERP has expired. If the check 145 finds that the time is longer than the AERP, then the timing of the next atrial pacing pulse is not changed, and the system delivers the next atrial pacing pulse as scheduled 139,167.

If the next atrial pacing pulse is delayed 149, and there is no atrial sense 144 before that time, then the energy of the delayed atrial pacing pulse may be increased 146 over a previously delivered pacing pulse to ensure capture. The energy of the delayed atrial pacing pulse may be increased, for example, by increasing the voltage and/or increasing the pulse width of the pacing pulse. A check 148 is then performed to see if an atrial evoked response is detected after delivery of the high energy atrial pacing pulse 147. This step determines if the current AERP is long enough. If the evoked response 148 to the high energy pace 147 is detected, then the AERP is sufficiently long, and the current AERP may be maintained. If there is no evoked response 148 to the high energy atrial pace, then the AERP is insufficient in length and is adjusted 165. The system paces or senses the ventricle 166 and waits 167 for the next A-A interval.

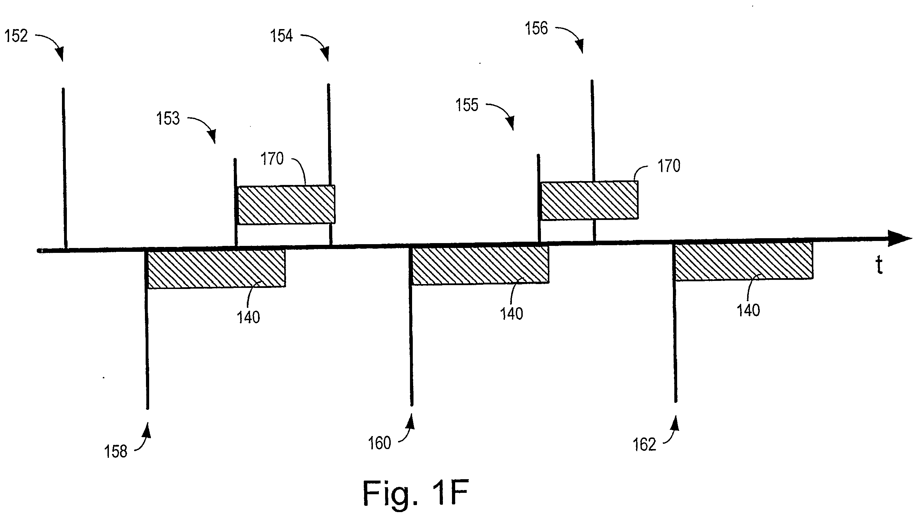

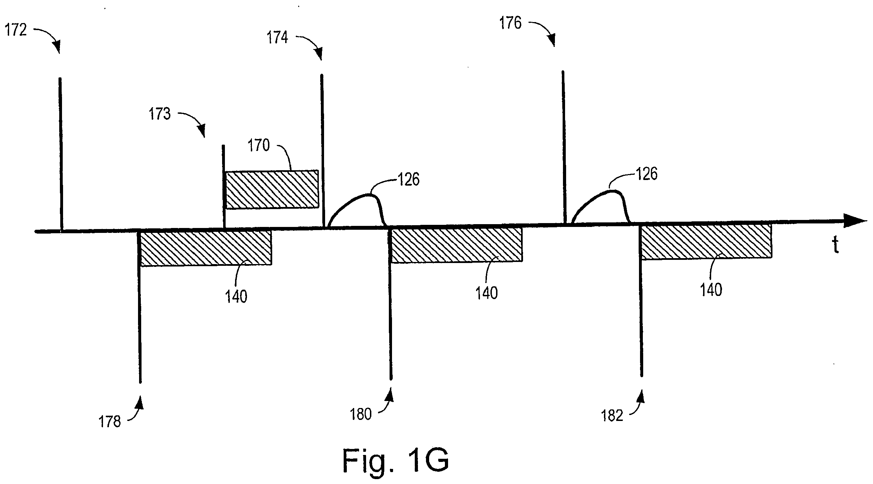

The operation of the retrograde management method described in connection with the flowchart of Figure 1E is illustrated in the graphs of Figures 1F and 1G. Figure 1F illustrates a pacing response graph of a pacemaker without backup pacing and no retrograde management. In the graph of Figure 1F, an atrial pace 152 is provided, but capture does not occur. A ventricular pace pulse 158 is delivered after an atrioventricular (AV) delay. The ventricular pace pulse 158 is followed by the PVARP 140. Retrograde conduction occurs, resulting in a retrograde P-wave 153 sensed during the PVARP 140. The PVARP prevents the retrograde P-wave from initiating an AV interval. However, with no retrograde management, the next scheduled atrial pace 154 is delivered during the atrial refractory period 170. The atrial pacing pulse 154 is ineffective, setting up another ventricular pace pulse 160 followed by the PVARP 140, and again a retrograde P-wave 155. This process may continue through an ineffective atrial pacing pulse 156 and a subsequent ventricular pacing pulse 162, resulting in repeated ineffective atrial paces and loss of AV synchrony.

Figure 1G illustrates a pacing response graph of a pacemaker with no backup pacing but having a method of retrograde management. In the graph of Figure 1G, an atrial pulse 172 is provided, but capture does not occur. A ventricular pace pulse 178 is provided after an atrioventricular delay. The ventricular pace pulse 178 is followed by the PVARP 140. Retrograde conduction occurs, resulting in retrograde P-wave 173 sensed during the PVARP 140. The pacemaker initiates an AERP 170 relative to the sensed retrograde P-wave 173. Due to the sensed retrograde P-wave 173, the pacemaker delays an atrial pace 174 for the next cardiac cycle until expiration of the AERP 170. Delaying the next scheduled atrial pace 174 until after expiration of the AERP 170 prevents the next scheduled atrial pace 174 from being delivered while the atrial tissue is refractory due to the retrograde P-wave 173. An evoked response signal 126 may be sensed after the atrial pace. AV synchrony and effective atrial pacing are maintained for atrial 176 and ventricular 180, 182 pacing pulses during subsequent cardiac cycles.

The capture verification and retrograde management processes described herein may be utilized in connection with pacing the left and/or right atria. Various embodiments involve using the same electrode combination for pacing and sensing. Other embodiments involve using an electrode combination for pacing that is different from the electrode combination used to sense the cardiac signal following pacing for capture verification. Employing different electrode combinations for pacing and sensing may enhance cardiac response determination. Further, the same or different electrode combinations may be utilized in sensing for an evoked response and in sensing for a retrograde P-wave.

The embodiments of the present system illustrated herein are generally described as being implemented in an implantable cardiac pacemaker/defibrillator (PD) that may operate in numerous pacing modes known in the art. Various types of single and multiple chamber implantable cardiac pacemaker/defibrillators are known in the art and may be used in connection with the atrial capture verification of the present invention. The present invention may be implemented in a variety of implantable or patient-external cardiac rhythm management devices, including dual chamber pacemakers, defibrillators, cardioverters, bi-ventricular pacemakers, cardiac resynchronizers, for example.

Although the present system is described in conjunction with an implantable cardiac pacemaker/defibrillator having a microprocessor-based architecture, it will be understood that the implantable cardiac pacemaker/defibrillator (or other device) may be implemented in any logic-based integrated circuit architecture, if desired.

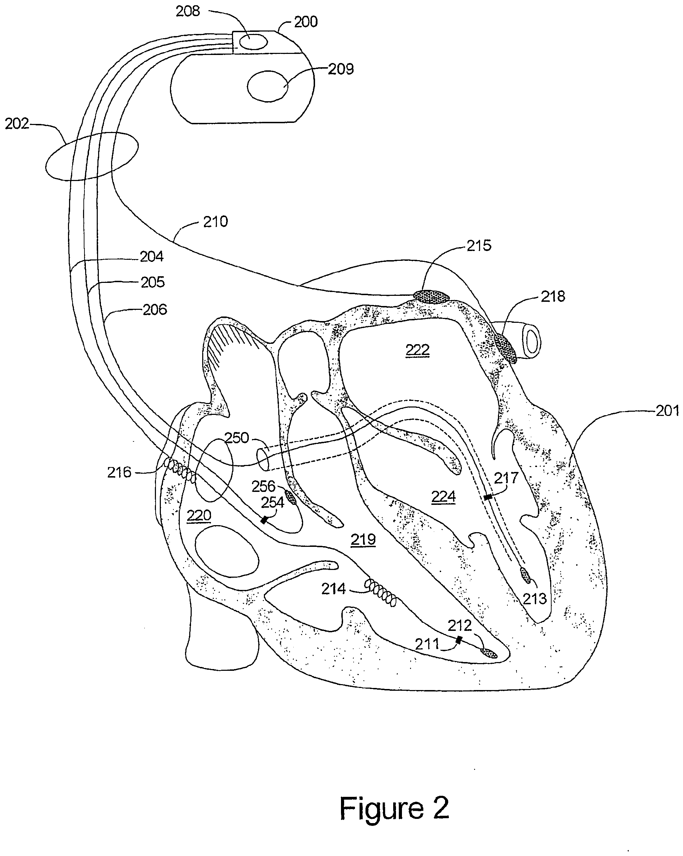

Referring now to Figure 2 of the drawings, there is shown a cardiac rhythm management system that may be used to implement capture verification and retrograde management methods. The cardiac rhythm management (CRM) system in Figure 2 includes a PD 200 electrically and physically coupled to a lead system 202. The housing and/or header of the PD 200 may incorporate one or more electrodes 208, 209 used to provide electrical stimulation energy to the heart and to sense cardiac electrical activity. The PD 200 may utilize all or a portion of the PD housing as a can electrode 209. The PD 200 may include an indifferent electrode 208 positioned, for example, on the header or the housing of the PD 200. If the PD 200 includes both a can electrode 209 and an indifferent electrode 208, the electrodes 208, 209 typically are electrically isolated from each other.

The lead system 202 is used to detect electric cardiac signals produced by the heart 201 and to provide electrical-energy to the heart 201 under certain predetermined conditions to treat cardiac arrhythmias. The lead system 202 may include one or more electrodes used for pacing, sensing, and/or defibrillation. In the embodiment shown in Figure 2, the lead system 202 includes an intracardiac right ventricular (RV) lead system 204, an intracardiac right atrial (RA) lead system 205, an intracardiac left ventricular (LV) lead system 206, and an extracardiac left atrial (LA) lead system 210. The lead system 202 of Figure 2 illustrates one embodiment that may be used in connection with the methodologies described herein. Other leads and/or electrodes may additionally or alternatively be used.

The lead system 202 may include intracardiac leads 204, 205, 206 implanted in a human body with portions of the intracardiac leads 204, 205, 206 inserted into a heart 201. The intracardiac leads 204, 205, 206 include various electrodes positionable within the heart for sensing electrical activity of the heart and for delivering electrical stimulation energy to the heart, for example, pacing pulses and/or defibrillation shocks to treat various arrhythmias of the heart.

As illustrated in Figure 2, the lead system 202 may include one or more extracardiac leads 210 having electrodes, e.g., epicardial electrodes, positioned at locations outside the heart for sensing and/or pacing one or more heart chambers.

The right ventricular lead system 204 illustrated in Figure 2 includes an SVC-coil 216, an RV-coil 214, an RV-ring electrode 211, and an RV-tip electrode 212. The right ventricular lead system 204 extends through the right atrium 220 and into the right ventricle 219. In particular, the RV-tip electrode 212, RV-ring electrode 211, and RV-coil electrode 214 are positioned at appropriate locations within the right ventricle 219 for sensing and delivering electrical stimulation pulses to the heart. The SVC-coil 216 is positioned at an appropriate location within the right atrium chamber 220 or a major vein leading to the right atrial chamber 220.

In one configuration, the RV-tip electrode 212 referenced to the can electrode 209 may be used to implement unipolar pacing and/or sensing in the right ventricle. Bipolar pacing and/or sensing in the right ventricle may be implemented using the RV-tip 212 and RV-ring 211 electrodes. In yet another configuration, the RV-ring 211 electrode may optionally be omitted, and bipolar pacing and/or sensing may be accomplished using the RV-tip electrode 212 and the RV-coil 214, for example. The right ventricular lead system 204 may be configured as an integrated bipolar pace/shock lead. The RV-coil 214 and the SVC-coil 216 are defibrillation electrodes.

The left ventricular lead 206 includes an LV distal electrode 213 and an LV proximal electrode 217 located at appropriate locations in or about the left ventricle 224 for pacing and/or sensing the left ventricle. The left ventricular lead 206 may be guided into the right atrium 220 of the heart via the superior vena cava. From the right atrium 220, the left ventricular lead 206 may be deployed into the coronary sinus ostium, the opening of the coronary sinus. The lead 206 may be guided through the coronary sinus to a coronary vein of the left ventricle 224. This vein is used as an access pathway for leads to reach the surfaces of the left ventricle that are not directly accessible from the right side of the heart. Lead placement for the left ventricular lead 206 may be achieved via subclavian vein access and a preformed guiding catheter for insertion of the LV electrodes 213,217 adjacent to the left ventricle.

Unipolar pacing and/or sensing in the left ventricle 224 may be implemented, for example, using the LV distal electrode 213 referenced to the can electrode 209. The LV distal electrode 213 and the LV proximal electrode 217 may be used together as bipolar sense and/or pace electrodes for the left ventricle. The left ventricular lead 206 and the right ventricular lead 204, in conjunction with the PD 200, may be used to provide cardiac resynchronization therapy such that the ventricles of the heart are paced substantially simultaneously, or in phased sequence, to provide enhanced cardiac pumping efficiency for patients suffering from heart failure.

The right atrial lead 205 includes a RA-tip electrode 256 and an RA-ring electrode 254 positioned at appropriate locations in the right atrium 220 for sensing and pacing the right atrium 220. In one configuration, the RA-tip 256 referenced to the can electrode 209, for example, may be used to provide unipolar pacing and/or sensing in the right atrium 220. In another configuration, the RA-tip electrode 256 and the RA-ring electrode 254 may be used to effect bipolar pacing and/or sensing.

Figure 2 illustrates one embodiment of a left atrial lead system 210. In this example, the left atrial lead 210 is implemented as an extracardiac lead with an LA distal electrode 218 and LA proximal electrode 215 positioned at an appropriate locations outside the heart 201 for sensing and pacing the left atrium 222. Unipolar pacing and/or sensing of the left atrium may be accomplished, for example, using the LA distal electrode 218 to the can 209 pacing vector. Bipolar pacing and/or sensing of the left atrium may be accomplished through the use of the LA distal electrode 218 and the LA proximal electrode 215.

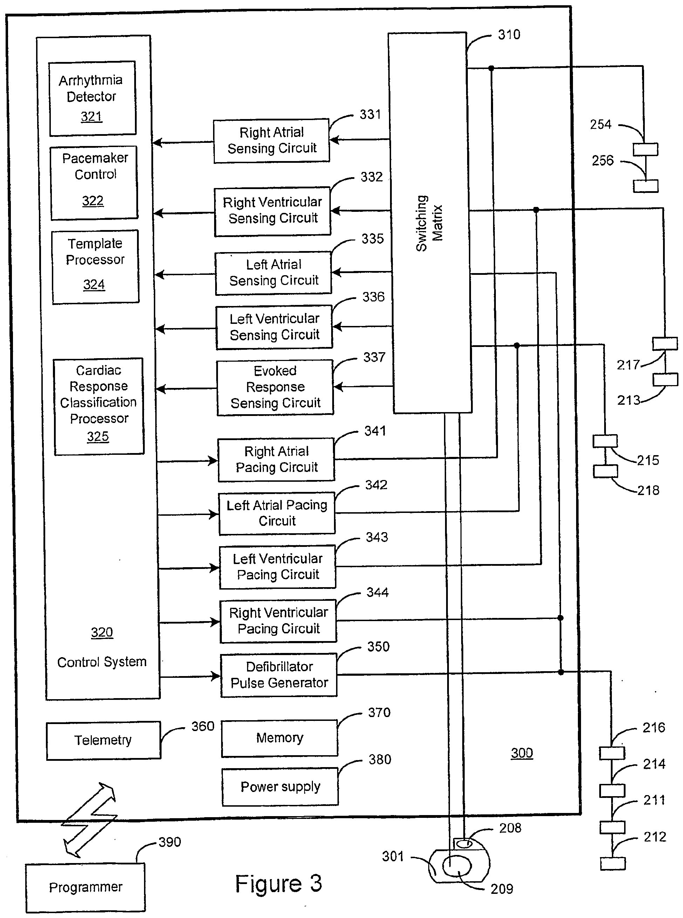

Referring now to Figure 3, there is shown an embodiment of a cardiac pacemaker/defibrillator 300 suitable for implementing an atrial capture verification and retrograde management methodologies. Figure 3 shows a cardiac pacemaker/defibrillator divided into functional blocks. It is understood by those skilled in the art that there exist many possible configurations in which these functional blocks can be arranged. The example depicted in Figure 3 is one possible functional arrangement. Other arrangements are also possible. For example, more, fewer or different functional blocks may be used to describe a cardiac pacemaker/defibrillator suitable for implementing the methodologies of the present invention. In addition, although the cardiac pacemaker/defibrillator 300 depicted in Figure 3 contemplates the use of a programmable microprocessor-based logic circuit, other circuit implementations may be utilized.

The cardiac pacemaker/defibrillator 300 depicted in Figure 3 includes circuitry for receiving cardiac signals from a heart and delivering electrical stimulation energy to the heart in the form of pacing pulses or defibrillation shocks. In one embodiment, the circuitry of the cardiac pacemaker/defibrillator 300 is encased and hermetically sealed in a housing 301 suitable for implanting in a human body. Power to the cardiac pacemaker/defibrillator 300 is supplied by an electrochemical battery 380. A connector block (not shown) is attached to the housing 301 of the cardiac pacemaker/defibrillator 300 to allow for the physical and electrical attachment of the lead system conductors to the circuitry of the cardiac pacemaker/defibrillator 300.

The cardiac pacemaker/defibrillator 300 may be a programmable microprocessor-based system, including a control system 320 and a memory 370. The memory 370 may store parameters for various pacing, defibrillation, and sensing modes, along with other parameters. Further, the memory 370 may store data indicative of cardiac signals received by other components of the cardiac pacemaker/defibrillator 300. The memory 370 may be used, for example, for storing historical electrogram (EGM) and therapy data. The historical data storage may include, for example, data obtained from long term patient monitoring used for trending or other diagnostic purposes. Historical data, as well as other information, may be transmitted to an external programmer unit 390 as needed or desired.

The control system 320 may cooperate with other components of the cardiac pacemaker/defibrillator 300 to control the operations of the cardiac pacemaker/defibrillator 300. In one example, the cardiac pacemaker/defibrillator 300 may incorporate a sensor for determining the patient's hemodynamic need. The sensor output may be utilized by the control system 320 to deliver pacing at a rate adapted to the activity level of the patient. In some implementations, the cardiac pacemaker/defibrillator 300 may include components of an accelerometer and/or a transthoracic impedance sensor for determining the activity level and/or respiration rate of the patient for determining hemodynamic need.

The control system 320 depicted in Figure 3 incorporates a cardiac response classification processor 325 for determining cardiac responses to pacing stimulation in accordance with various embodiments of the present invention. The control system 320 may include additional functional components including a pacemaker control circuit 322, an arrhythmia detector 321, and a template processor 324, along with other components for controlling the operations of the cardiac pacemaker/defibrillator 300.

Telemetry circuitry 360 may be implemented to provide communications between the cardiac pacemaker/defibrillator 300 and an external programmer unit 390. In one embodiment, the telemetry circuitry 360 and the programmer unit 390 communicate using a wire loop antenna and a radio frequency telemetric link, as is known in the art, to receive and transmit signals and data between the programmer unit 390 and the telemetry circuitry 360. In this manner, programming commands and other information may be transferred to the control system 320 of the cardiac pacemaker/defibrillator 300 from the programmer unit 390 during and after implant. In addition, stored cardiac data pertaining to capture threshold, capture detection and/or cardiac response classification, for example, along with other data, may be transferred to the programmer unit 390 from the cardiac pacemaker/defibrillator 300.

In the embodiment of the cardiac pacemaker/defibrillator 300 illustrated in Figure 3, electrodes RA-tip 256, RA-ring 254, RV-tip 212, RV-ring 211, RV-coil 214, SVC-coil 216, LV distal electrode 213, LV proximal electrode 217, LA distal electrode 218, La proximal electrode 215, indifferent electrode 208, and can electrode 209 are coupled through a switch matrix 310 to sensing circuits 331-337.

A right atrial sensing circuit 331 serves to detect and amplify electrical signals from the right atrium of the heart. Unipolar sensing may be implemented, for example, by sensing voltages developed between the RA-tip 256 and the can electrode 209. Outputs from the right atrial sensing circuit are coupled to the control system 320.

A right ventricular sensing circuit 332 serves to detect and amplify electrical signals from the right ventricle of the heart. Right ventricular cardiac signals sensed through use of the RV-tip 212 electrode are right ventricular near-field signals and are denoted RV rate channel signals. A bipolar RV rate channel signal may be sensed as a voltage developed between the RV-tip 212 and the RV-ring 211. Alternatively, bipolar sensing in the right ventricle may be implemented using the RV-tip electrode 212 and the RV-coil 214. Unipolar rate channel sensing in the right ventricle may be implemented, for example, by sensing voltages developed between the RV-tip 212 and the can electrode 209.

Right ventricular cardiac signals sensed through use of defibrillation electrodes may be far-field signals, also referred to as RV morphology or RV shock channel signals. More particularly, a right ventricular shock channel signal may be detected as a voltage developed between the RV-coil 214 and the SVC-coil 216. A right ventricular shock channel signal may also be detected as a voltage developed between the RV-coil 214 and the can electrode 209. In another configuration the can electrode 209 and the SVC-coil electrode 216 may be electrically shorted and a RV shock channel signal may be detected as the voltage developed between the RV-coil 214 and the can electrode 209/SVC-coil 216 combination.

Left atrial cardiac signals may be sensed through the use of one or more left atrial electrodes 215, 218, which maybe configured as epicardial electrodes. A left atrial sensing circuit 335 serves to detect and amplify electrical signals from the left atrium of the heart. Bipolar sensing and/or pacing in the left atrium may be implemented, for example, using the LA distal electrode 218 and the LA proximal electrode 215. Unipolar sensing and/or pacing of the left atrium may be accomplished, for example, using the vector from the LA distal electrode 218 to can electrode 209 or the LA proximal electrode 215 to can electrode 209.

A left ventricular sensing circuit 336 serves to detect and amplify electrical signals from the left ventricle of the heart. Bipolar sensing in the left ventricle may be implemented, for example, by sensing voltages developed between the LV distal electrode 213 and the LV proximal electrode 217. Unipolar sensing may be implemented, for example, by sensing voltages developed between the LV distal electrode 213 or the LV proximal electrode 217 to the can electrode 209.

Optionally, an LV coil electrode (not shown) may be inserted into the patient's cardiac vasculature, e.g., the coronary sinus, adjacent the left heart. Signals detected using combinations of the LV electrodes, 213, 217, LV coil electrode (not shown), and/or can electrodes 209 may be sensed and amplified by the left ventricular sensing circuitry 336. The output of the left ventricular sensing circuit 236 is coupled to the control system 320.

The outputs of the switching matrix 310 may be operated to couple selected combinations of electrodes 211, 212, 213, 214, 216, 217, 218, 254, and 256 to an evoked response sensing circuit 337. The evoked response sensing circuit 337 serves to sense and amplify voltages developed using various combinations of electrodes for cardiac response classification in accordance with embodiments of the invention.

In the embodiments described herein, various combinations of pacing and sensing electrodes may be utilized in connection with pacing and sensing the cardiac signal following the pace pulse to classify the cardiac response to the pacing pulse. For example, in some embodiments, a first electrode combination is used for pacing a heart chamber and a second electrode combination is used to sense the cardiac signal following pacing. In other embodiments, the same electrode combination is used for pacing and sensing. Further, the electrodes used to sense for the retrograde P-wave may be different or the same as the electrodes used to sense for the atrial evoked response.

The pacemaker control circuit 322, in combination with pacing circuitry for the left atrium, right atrium, left ventricle, and right ventricle may be implemented to selectively generate and deliver pacing pulses to the heart using various electrode combinations. The pacing electrode combinations may be used to effect bipolar or unipolar pacing of the heart chambers as described herein.

As described herein, bipolar or unipolar pacing pulses may be delivered to a heart chamber using one of the pacing vectors as described above. The electrical signal following the delivery of the pacing pulses may be sensed through various sensing vectors coupled through the switch matrix 310 to the cardiac response classification processor 325 and used to classify the cardiac response to pacing.

The switching matrix 310 may be arranged to provide connections to various configurations of pacing and defibrillation electrodes. The outputs of the switching matrix 310 may be coupled to an evoked response (ER) sensing circuit 337 that serves to sense and amplify cardiac signals detected between the selected combinations of electrodes. The detected signals are coupled through the ER amplifier 337 to a cardiac response classification processor 325. The cardiac response classification processor 325 includes circuitry configured to determine the cardiac response to a pacing stimulation. The presence or absence of an evoked response may be determined based on the amplitude, peak value, peak timing, and/or other morphological features of the cardiac signal sensed following the pacing pulse in accordance with embodiments of the invention.

In one implementation, the cardiac pacemaker/defibrillator 300 may utilize the evoked response channel 337 to sense for the atrial evoked response (AER) as described herein. The cardiac pacemaker/defibrillator 300 may utilize the right atrial sensing channel 331 to sense for retrograde P-waves in the right atrium. The cardiac pacemaker/defibrillator 300 may utilize the left atrial sensing channel 335 to sense for retrograde P-waves in the left atrium.

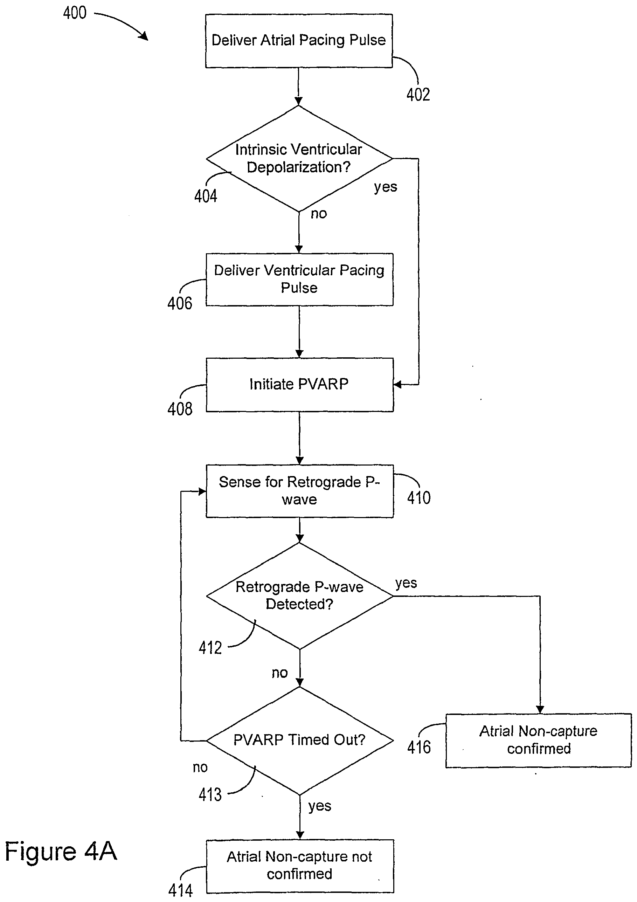

Figure 4A is a flowchart further describing methods 400 of capture detection based on retrograde conduction. An atrial pacing pulse 402 is delivered, and a check 404 is made to detect intrinsic ventricular depolarization. If the check 404 finds no ventricular depolarization, a ventricular pacing pulse 406 is delivered. A PVARP 408 is initiated following the ventricular pacing pulse or the intrinsic ventricular depolarization. After initiating PVARP 408, the system senses 410 for a retrograde P-wave. If the retrograde P-wave is detected 412, during 413 PVARP, then atrial non-capture 416 is confirmed. If the retrograde P-wave is not detected 412 during 413 PVARP, atrial non-capture 414 is not confirmed.

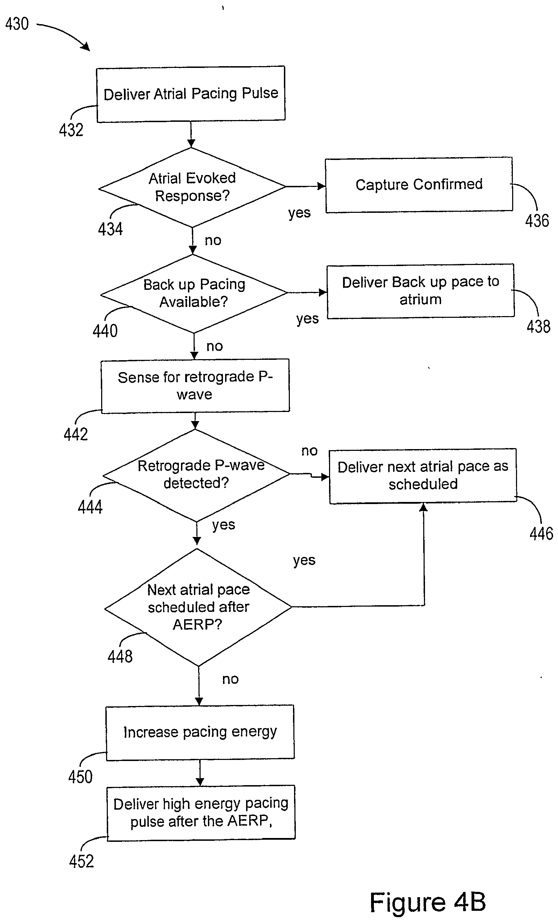

Figure 4B is a flowchart of a method 430 of retrograde management. Atrial pacing pulse 432 is delivered, and a check 434 is made to detect an atrial evoked response. If the check 434 finds no atrial evoked response, and backup pacing 440 is not available, then a sense 442 is done for a retrograde P-wave. If a retrograde P-wave is detected 444, and the next atrial pulse is scheduled 448 after AERP, the next atrial pulse is delivered 446 as scheduled. If the next atrial pace is not scheduled 448 after AERP, then the pacing energy is increased 450 and a high energy pacing pulse is delivered 452 after the AERP. If, at the check 434 for atrial evoked response, an evoked response is detected, then capture is confirmed 436. If the atrial evoked response is not detected at check 434, but backup pacing 440 is available, then backup pacing is delivered 438 to the atrium. If a retrograde P-wave is not detected 444, the next atrial pace 446 may be delivered as scheduled.

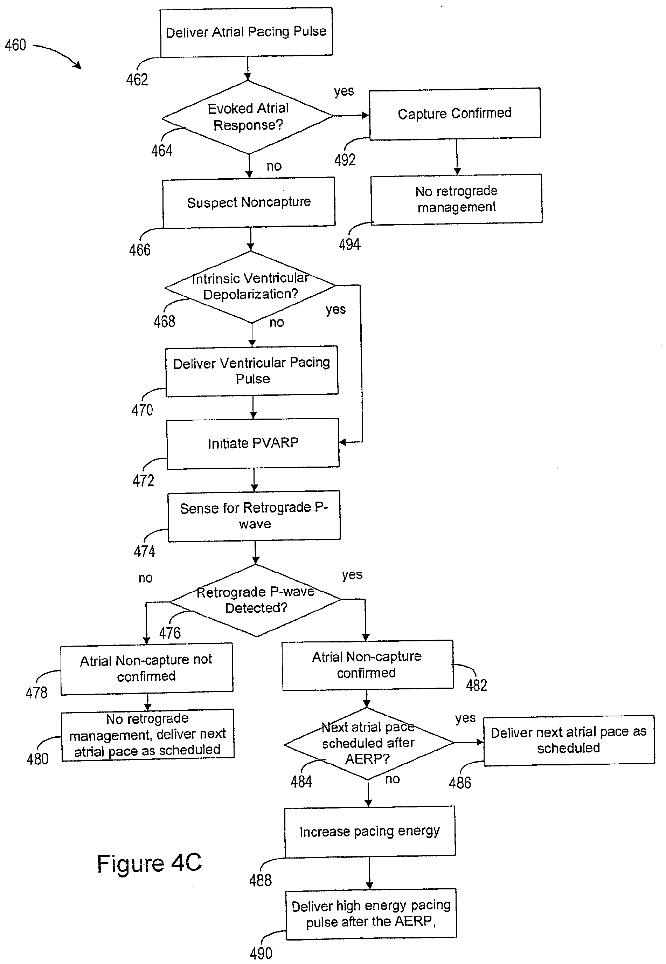

Figure 4C is a flowchart of a method 460 of capture verification and retrograde management. Atrial pacing pulse 462 is delivered, and a check 464 is made to detect an atrial evoked response. If there is 464 a detected atrial evoked response, then capture is confirmed 492, and no retrograde management 494 is used. If there is no 464 detected atrial evoked response, then non-capture is suspected 466, and a check for intrinsic ventricular depolarization 468 is performed. If the check 468 finds no ventricular depolarization, a ventricular pacing pulse 470 is delivered. If the check 468 finds a ventricular depolarization, a PVARP 472 is initiated without the ventricular pacing pulse 470. After initiating PVARP 472, a sense 474 is made for a retrograde P-wave. If the retrograde P-wave is detected 476, atrial non-capture 482 is confirmed, and a check 484 is performed to see if the next atrial pace is scheduled after the AERP. If the check 484 determines that the pace is scheduled after the AERP, then the next atrial pulse is delivered 486 as scheduled. If the check 484 determines that the pace is not scheduled after the AERP, then the pacing energy is increased 488 and the higher energy pulse is delivered 490 after the AERP. If the retrograde P-wave is not detected at 476, atrial non-capture 478 is not confirmed, and no retrograde management 480 is used, so the next atrial pulse is delivered as scheduled.

Embodiments of the invention involve methodologies used for capture detection. The methods and systems described herein advantageously utilize the stable morphology of the captured response signal to discriminate between capture and fusion beats. The variability in the timing of a particular feature of a captured response signal may be determined with some degree of confidence. The timing variability of a captured response signal feature may be used in connection with capture verification as exemplified in the embodiments described herein.

Capture of a heart chamber may be detected by analyzing the cardiac signal of the heart chamber following a pacing pulse. The cardiac signal is sensed during a classification interval initiated after a blanking period that follows the pacing pulse. The cardiac signal sensed within the classification interval is used to determine the cardiac response to the pacing pulse. For example, one or more features, samples, or morphological characteristics of the cardiac signal sensed within the classification interval may be compared to a template or other criteria characterizing a particular type of cardiac response to pacing. For example, the template may characterize a captured response, a non-captured response, or a fusion beat. If the cardiac signal sensed during the classification interval is consistent with the template, then the cardiac response to the pacing pulse is classified as the particular type of pacing response represented by the template.

A cardiac signal may be considered to be consistent with a template if the features, samples, or morphological characteristics of the cardiac signal are determined to be sufficiently similar to corresponding template features, samples, or morphological characteristics. If a cardiac signal is sufficiently similar to a template representative of a particular type of cardiac response, then the cardiac signal may be classified as the particular type of cardiac response. Some embodiments are directed to methods and systems for implementing an adaptable cardiac response classification interval useful for classifying a cardiac response to a pacing pulse. The classification interval may be adapted, for example, using statistical parameters associated with one or more features of the cardiac response signal. The adaptable classification interval may be utilized to detect capture and/or to discriminate between two or more of a captured response, non-captured response, and a fusion beat.

The adaptable classification interval represents the period of time following a pacing pulse that a cardiac signal feature indicative of a particular type of cardiac pacing response, e.g., capture, is most likely to be detected. The cardiac signal sensed during the adaptable interval is used to determine the cardiac pacing response. An adaptable classification interval may be individualized for each patient, thereby reducing cross patient variance. The adaptable classification interval may be particularly useful in discriminating between fusion and capture for atrial pacing because atrial fusion management cannot rely on shortening the AV delay to enforce pacing and avoid fusion as in ventricular fusion management.

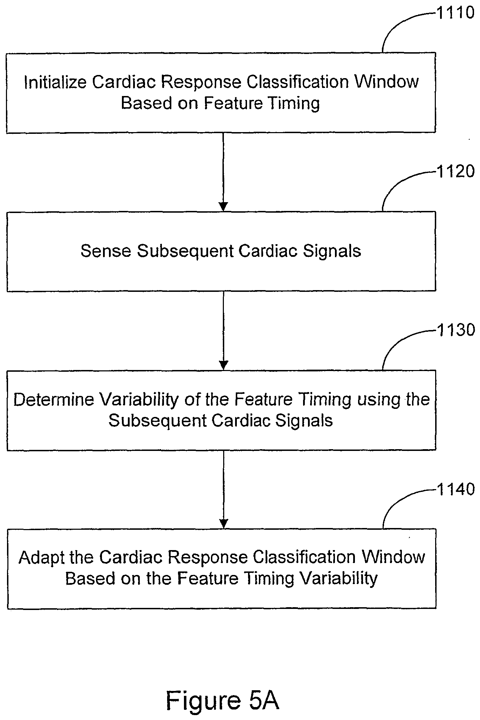

A method of initializing and adapting a cardiac response classification interval is illustrated in the flowchart of Figure 5A. The classification interval comprises a period of time during which a particular feature of the cardiac signal is most likely to be detected for a particular type of cardiac pacing response. The classification interval may be initialized 1110 based on the timing of a cardiac signal feature, such as a cardiac signal feature associated with a captured response. The cardiac signal feature may comprise, for example, a positive peak, negative peak, or other morphological characteristic of the cardiac signal.

The duration of the initial classification interval may have a predetermined length. In some embodiments, the timing of the cardiac signal feature may be used to define the central point of the classification interval. In other embodiments, classification interval may be otherwise temporally oriented with respect to the cardiac signal feature. In one scenario, the timing of the feature used for the initial classification interval is determined based on a cardiac response template generated during a template initialization procedure. The template initialization procedure may be performed to acquire a template comprising one or more features that characterize a captured response.

Following initialization of the classification interval, one or more subsequent cardiac signals associated with the type of cardiac response, e.g., a captured response, are sensed 1120. The one or more subsequent cardiac signals may be sensed, for example, following pacing pulses delivered in connection with the patient's prescribed pacing regimen, or during other processes involving cardiac pacing and capture detection. The timing variability of the cardiac signal feature is determined 1130 using historic and/or the subsequent cardiac signals. The duration of the classification interval is adapted 1140 based on the timing variability.

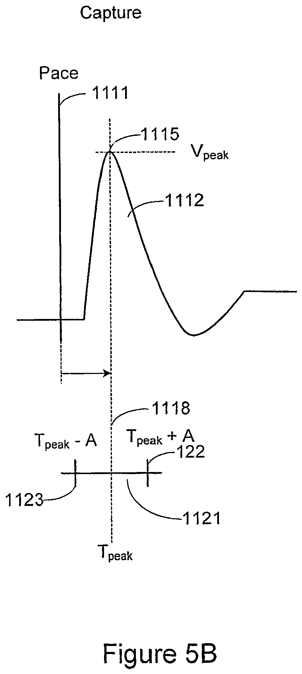

Figure 5B graphically illustrates the orientation of the classification interval with respect to a selected cardiac signal feature, which in this example comprises the positive peak of the cardiac signal. In this embodiment, the classification interval 1121 of duration 2 x A is initialized based on the timing 1118 of the peak 1115 of a captured response signal 1112 that follows a pacing pulse 1111, as illustrated by the graph of Figure 5B. The peak timing 1118 marks the center of the classification interval 1121.

Following initialization, one or more paces subsequent to pace 1111 are delivered and the system senses the cardiac signals following delivery of the subsequent paces. If the subsequent signals indicate capture, then the peak amplitude timing of each of the subsequent captured response signals is detected. The variability of the peak amplitude timing of the captured response signals is determined. The peak amplitude timing variability is used to update or adapt the upper and lower bounds 1122, 1123 of the initial classification interval 1121.

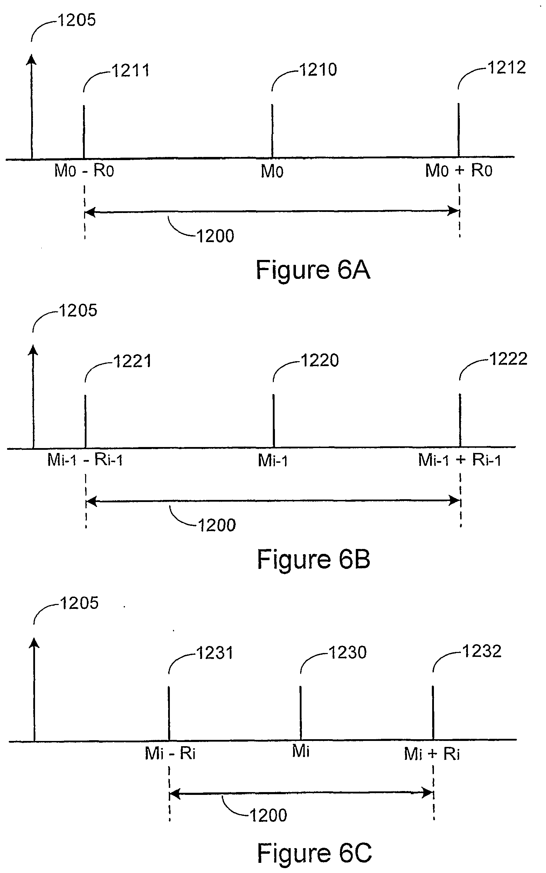

An embodiment involving an adaptable classification interval is illustrated in Figures 6A-6C. A captured response template comprising a peak amplitude and a peak amplitude timing, T₀, is acquired during an initialization process. The classification interval 1200 is initialized as an interval of time following a pacing pulse 1205. The initial cardiac response classification interval 1200 uses T₀ as the initial center point 1210, M₀, of the classification interval 1200. A predetermined range, R₀, is selected and used as the initial range for the initial classification interval. Thus, the initial cardiac response classification interval has an initial midpoint 1210, M₀, an upper range limit 1211 of M₀ + R₀ and a lower range limit 1212 of M₀ - R₀.

Figures 6B and 6C illustrate the adaptation of the classification interval. Figure 6B illustrates the classification interval prior to adaptation. The center point 220 of the classification designated Mᵢ₋₁. The upper range limit 1221 of the classification interval is designated Mᵢ₋₁ + Rᵢ₋₁ and the lower range limit 1222 is designated Mᵢ₋₁ - Rᵢ₋₁. Figure 6C illustrates the cardiac response classification interval after the next paced beat, referred to as the i^{th} beat. The peak amplitude time, Tᵢ, of the i^{th} beat is determined. If Tᵢ falls into the range of [Mᵢ₋₁ - Rᵢ₋₁, Mᵢ₋₁ + Rᵢ₋₁] then the 1^{th} beat is considered to be a non-fusion, captured beat and Tᵢ is used to adapt the classification interval. Otherwise, the i^{th} beat is considered fusion and the range will not be updated using the information from that beat.

In one embodiment, Tᵢ is used to generate a new central point 1230, Mᵢ, a new lower range 1231, Mᵢ - Rᵢ, and a new upper range 1232, Mi + Rᵢ, of an adapted classification interval as illustrated in Figure 6C. A new value for Mᵢ may be determined, for example, based on a statistical function of the peak timing associated with the previous capture beats. In one example, a new value for Mᵢ comprises the average of the peak amplitude timing values of all the previous captured beats, Tₖ, where k = 1, 2, 3, ..., i) Other statistical functions (weighted average, median value) may alternatively be used to calculate Mᵢ. A new value for Rᵢ may be calculated, for example, as f*SD, where f is a constant, and SD is the standard deviation of the peak amplitude timings of all previous captured beats, Tₖ.

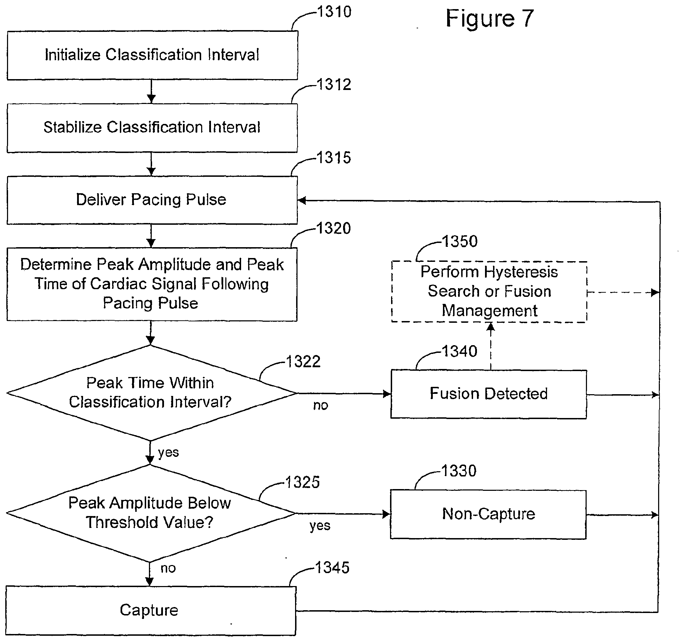

Figure 7 is a flowchart illustrating a method of classifying a cardiac response to a pacing pulse using an adapted classification interval.

The classification interval is initialized 1310 to an initial center point and range. The initial classification interval is adjusted 1312 for stability. During the stabilization process, the cardiac response classification interval including, for example, the midpoint, upper bound and/or lower bound of the classification interval is modified based on one or more captured beats. In one implementation, the classification interval may be adjusted using a predetermined number of captured beats. In another implementation, the classification interval may be adjusted until predetermined stability criteria are achieved.

Following delivery 1315 of a pacing pulse to a heart chamber, the peak amplitude and peak time of the cardiac signal sensed in the heart chamber is determined 320. If the peak time is beyond 1322 the classification interval, the cardiac pacing response is classified 1340 as fusion. If the peak time falls within the classification interval, then the cardiac signal amplitude is checked 1325.

If the peak amplitude is greater than or equal to 1325 the threshold value, then the cardiac pacing response is classified 1345 as capture. If the peak amplitude is less than 1325 the threshold value, then the cardiac pacing response is classified as non-capture.

If fusion is detected 1340, then the system may optionally initiate 1350 a fusion management or a hysteresis search, a process that involves lengthening a pacing interval to encourage intrinsic activity to occur before the next pacing (in the application of beat-to-beat capture verification) or shortening a pacing interval to promote the next pacing (in the application of threshold testing). For example, in AAI or VVI pacing, the pacing escape interval (A-A interval or V-V interval) for the next cardiac cycle after fusion is detected may be lengthened to allow intrinsic activity to occur. In DDD pacing, the atrioventricular delay may be lengthened after detecting fusion to promote intrinsic activity.

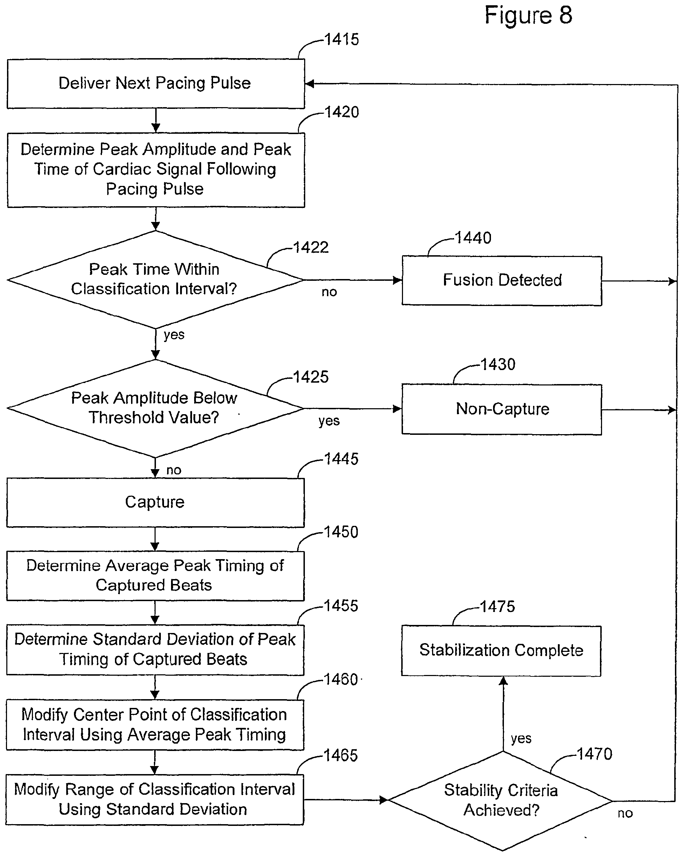

If fusion is detected 1340, then the system may optionally initiate 1350 a fusion management process. Fusion management may involve, for example, sensing prior to delivery of a pacing pulse and delaying the pace if the sensed amplitude exceeds a sensing level threshold. A rise in the amplitude of the cardiac signal may indicate the presence of intrinsic activity. If no intrinsic activity is detected, a backup pace is delivered, the backup pace having sufficient energy to assure capture. Methods and systems involving fusion management techniques, aspects of which may be incorporated in the embodiments described herein, are discussed in commonly owned U.S. Patent 6,038,474. Figure 8 is a flowchart illustrating a method of stabilizing the cardiac response classification interval that may be used, for example, at block 1312 of Figure 7. During this process, the cardiac response interval is adapted until stability is achieved. A pacing pulse is delivered 1415 to the heart chamber. Following delivery 1415 of the pacing pulse, the peak amplitude and peak timing of the cardiac signal sensed in the heart chamber are determined 1420. If the peak time is beyond 1422 the classification interval, the cardiac pacing response is classified 1440 as fusion. If the peak time falls within 1422 the classification interval, then the cardiac signal amplitude is checked 1425.

If the peak amplitude is greater than or equal to 1425 the threshold value, then the cardiac pacing response is classified 1445 as capture. If the peak amplitude is less than 1425 the threshold value, then the cardiac pacing response is classified 1430 as non-capture.

If the cardiac response to pacing is classified 1445 as capture, the peak timing of the cardiac signal is used to adapt the classification interval. The average and standard deviation of the peak timing of the captured beats is determined 1450, 1455. The center point the classification interval is modified 1460 using the average peak timing. The range of the classification interval is modified 1465 using the standard deviation of the peak timing. If a predetermined stability criteria have been achieved 1470, then stabilization is complete 1475. Otherwise, the process continues until the stabilization criteria is met, or until the process times out. In one implementation, the stability criteria may involve, for example, the use of a predetermined number of beats to adapt the classification interval. In another implementation, adaptation of the classification interval may continue until a predetermined stability criteria is achieved, e.g., a variability of the center point and/or range below a predetermined value, such as if the standard deviation divided by the median value is below about 0.5.

By way of example, the present invention may be employed to determine the cardiac response in connection with threshold testing to determine the optimal energy for pacing. Determination of the optimal pacing energy may be implemented, for example, by a capture threshold testing procedure. Additionally, the cardiac response classification interval may be employed in connection with an automatic capture verification process used to monitor pacing responses on a beat-by-beat basis. Automatic capture verification may be used to control back up pacing when a pace pulse delivered to the heart fails to evoke a captured response. These and other applications may be enhanced by employment of the systems of the present invention.

Those skilled in the art will appreciate that reference to a capture threshold testing procedure indicates a method of determining the capture threshold in one of left atrium, right atrium, left ventricle, and right ventricle. In such a procedure, the pacemaker, automatically or upon command, initiates a search for the capture threshold of the selected heart chamber. The capture threshold is defined as the lowest pacing energy that consistently captures the heart.

In one example of an automatic capture threshold procedure, the pacemaker delivers a sequence of pacing pulses to the heart and detects the cardiac responses to the pace pulses. The energy of the pacing pulses may be decreased in discrete steps until a predetermined number of loss-of-capture responses occur. The pacemaker may increase the stimulation energy in discrete steps until a predetermined number of capture responses occur to confirm the capture threshold. A capture threshold test may be performed using cardiac response classification methods of the present invention.

Other procedures for implementing capture threshold testing may be utilized. In one example, the pacing energy may be increased in discrete steps until capture is detected. In another example, the pacing energy may be adjusted according to a binomial search pattern.

Capture threshold determination is distinguishable from automatic capture detection, a procedure that typically occurs on a beat-by-beat basis during pacing. Automatic capture detection verifies that a delivered pace pulse results in a captured response. When a captured response is not detected following a pace pulse, the pacemaker may deliver a back up safety pace to ensure consistent pacing. The back up pace may be delivered, for example, about 70-80 ms after the initial pace pulse. If a predetermined number of pace pulses delivered during normal pacing do not produce a captured response, the pacemaker may initiate a capture threshold test to determine the capture threshold. Alternatively, if a predetermined number of pacing pulses do not produce a captured response, the pacemaker may adjust the pacing energy for the next pacing pulse. Automatic capture detection and back up pacing may be implemented using the adaptable classification intervals and/or the cardiac response classification processes of the present invention.

The pacing pulse may be delivered to any heart chamber and the cardiac response of the heart chamber to pacing may be determined by evaluating the cardiac signal sensed in the chamber following the pacing pulse. For example, the pacing stimulation may be delivered to one of the right ventricle, the left ventricle, the right atrium, and the left atrium. Various embodiments of the invention involve using the same electrode combination for pacing and sensing. Other embodiments involve using an electrode combination for pacing that is different from the electrode combination used to sense the cardiac signal following pacing. Employing different electrode combinations for pacing and sensing may enhance cardiac response classification. For example, using different electrode combinations for pacing and sensing may facilitate detection of capture, and/or may enhance discrimination between captured beats and fusion beats.

Embodiments described herein may involve the initialization and use of an adaptable classification interval or may utilize a classification interval of a predetermined duration Classification of the cardiac pacing response may be based on the variability of one or more features of a cardiac signal detected within the classification interval. Selected features of the cardiac signal may be tracked beat-to-beat. The variability of the selected features may be used to discriminate between capture and fusion beats, for example.

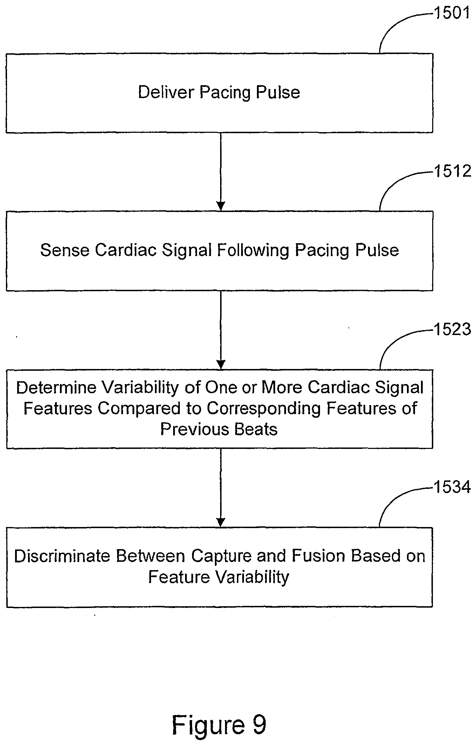

Figures 9, 10, and 11 illustrate capture detection methodologies based on the variability of selected features of the cardiac signal. If the variability of selected features, such as peak amplitude or peak timing, is determined to be beyond a variability threshold, then the cardiac beat is classified as fusion. If the variability of the selected features is within the variability threshold, then the system may further classify the cardiac response, such as a captured beat or a non-captured beat. The variability threshold may be determined, for example, based on a standard deviation or other statistical function associated with a selected feature of previous cardiac signals representative of a particular type of cardiac response. The cardiac response classification methodologies illustrated in Figures 9, 10, and 11 are particularly useful in connection with discriminating between capture and fusion beats in either atrial or ventricular chamber.

The invention may be used in conjunction with pacing or capture threshold testing and/or with beat-to-beat automatic capture verification. Figure 9 is a flowchart illustrating the process of discriminating between capture and fusion beats. A pacing pulse is delivered 1501 and the cardiac signal following the pacing pulse is sensed 1512. One or more features of the cardiac signal are detected. The variability of the one or more features of the cardiac signal compared to the corresponding features of previous cardiac beats is determined 1523. The system discriminates 1534 between capture and fusion beats based on the variability of the cardiac signal features.

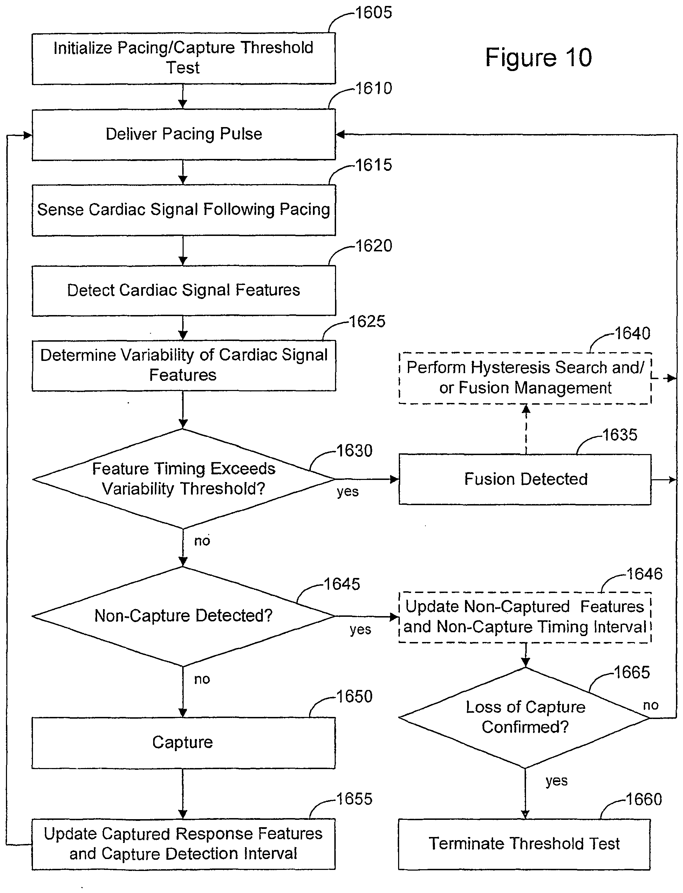

The flowchart of Figure 10 further illustrates cardiac response determination process. During an initialization phase 1605, the system delivers a series of pacing pulses and tracks the variability of cardiac signal features. A variability threshold for each tracked feature is determined based on the signals detected during the initialization phase.

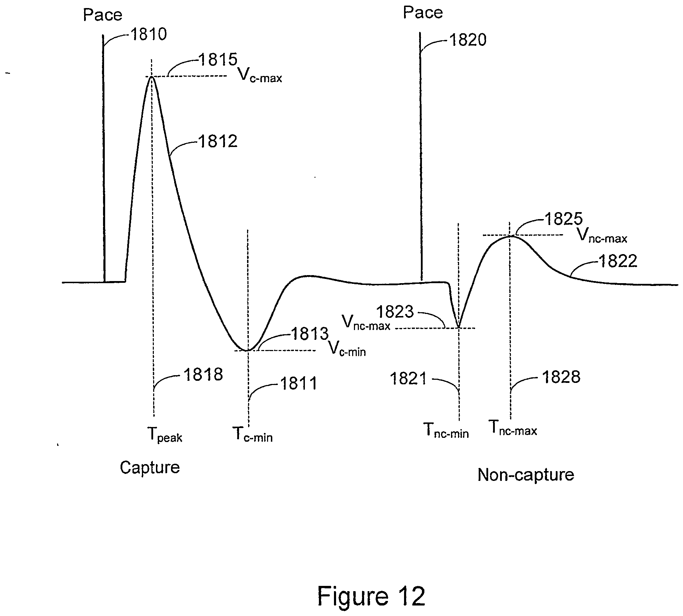

A pacing pulse 1610 is delivered to a heart chamber and the cardiac signal following the pacing pulse is sensed 1615. The cardiac signal features of the cardiac signal are detected 1620. For example, with reference to the graph of Figure 12, if the cardiac signal is a non-captured signal 1822, then the detected features of the cardiac signal may include one or more of the maximum peak amplitude 1825, V_{nc-max}, the maximum peak amplitude timing 1828, T_{nc-max}, the minimum peak amplitude 1823, V_{nc-min}, and/or the minimum peak amplitude timing 1821, T_{nc-min}.

If the cardiac signal is a captured response 1812, then the detected features of the cardiac signal may include one or more of the maximum peak amplitude 1815, V_{c-max}, the maximum peak amplitude timing 1818, T_{c-max}, the minimum peak amplitude 1813, V_{c-min}, and/or the minimum peak amplitude timing 1811, T_{c-min}.

Returning to Figure 10, the system determines 1625 the variability of the of the detected cardiac signal features. If the feature timing variability exceeds 1630 a variability threshold then fusion is detected 1635. In this scenario, a hysteresis search or fusion management process may be implemented 1640 as described with reference to Figure 7.

If the feature timing variability does not exceed 1630 the variability threshold, the system checks to determine whether or not the pacing pulse produced capture. If non-capture is determined 1645, then the cardiac signal may be used to update 1646 one or more variability thresholds respectively associated with features of the non-captured response. For example, the non-captured peak timing may be used to update a non-captured peak timing variability threshold. In another example, a non-captured peak amplitude may be used to update a non-captured amplitude variability threshold.

If loss of capture is confirmed 1665, then the capture threshold is determined and the test is complete and terminated 1660. Loss of capture may be confirmed if x out of y beats are determined to be non-captured beats. In one example, loss of capture may be confirmed if about 2 out of about 4 beats are non-captured beats.

If the beat is determined 1645 to be a captured beat, then the cardiac signal is used to update 1655 the capture detection interval and/or one or more variability thresholds respectively associated with features of the captured response. For example, the captured response peak timing may be used to update the range and/or midpoint of the classification interval. The peak timing of the capture response signal may be used to update the captured response peak timing variability threshold. The peak amplitude of the captured response signal may be used to update a captured amplitude variability threshold.

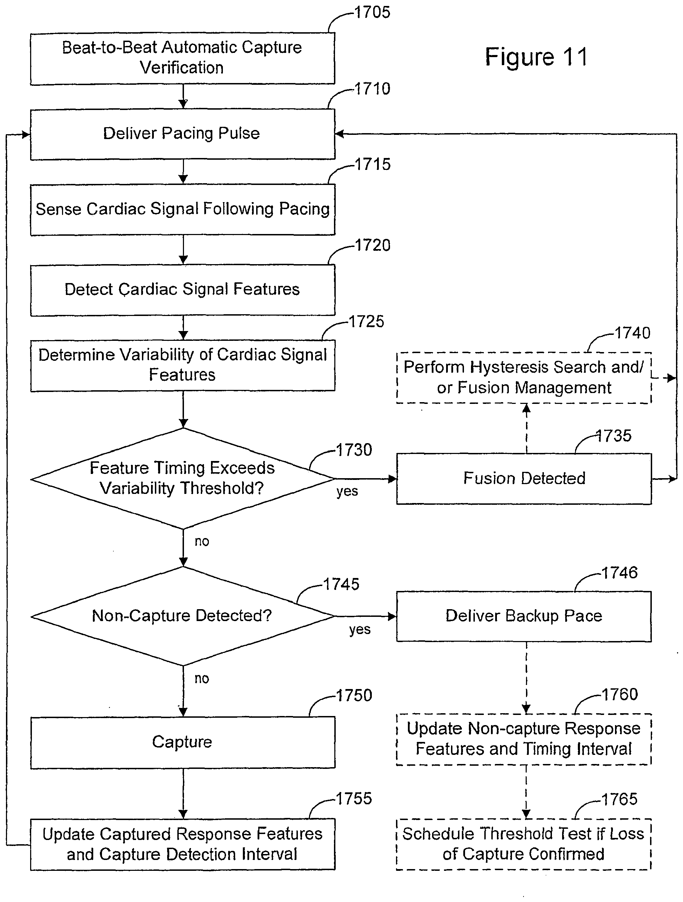

The flow chart of Figure 11 illustrates a capture detection methodology used for beat-to-beat automatic capture verification 1705. A pacing pulse 1710 is delivered to the heart chamber and the cardiac signal following the pacing pulse is sensed 1715. The cardiac signal features of the cardiac signal are detected 1720. For example, with reference to the graph of Figure 12, if the cardiac signal is a non-captured signal 1822, then the detected features of the cardiac signal may include one or more of the maximum peak amplitude 1825, V_{nc-max}, the maximum peak amplitude timing 1828, T_{nc-max}, the minimum peak amplitude 1823, V_{nc-min}, and/or the minimum peak amplitude timing 1821, T_{nc-min}.

If the cardiac signal is a captured response 1812, then the detected features of the cardiac signal may include one or more of the maximum peak amplitude 1815, V_{c-max}, the maximum peak amplitude timing 1818, T_{c-max}, the minimum peak amplitude 1813, V_{c-min} and/or the minimum peak amplitude timing 1811, T_{c-min}.

The system determines 1725 the variability of the of the detected cardiac signal features. If the feature timing variability exceeds 1730 a variability threshold then fusion is detected 1735. In this scenario, a hysteresis search or fusion management process may be implemented 1740 as previously described with reference to Figure 7.

If the feature timing variability does not exceed 1730 the variability threshold, the system checks to determine whether or not the pacing pulse produced capture. If non-capture is determined 1745, then a backup pace is typically delivered 1746 at an energy level that assures capture. The non-captured cardiac signal may be used to update 1760 one or more variability thresholds respectively associated with features of the non-captured response. For example, the non-captured peak timing may be used to update a non-captured peak timing variability threshold. In another example, a non-captured peak amplitude may be used to update a non-captured amplitude variability threshold. If loss of capture is confirmed, then a capture threshold test may optionally be scheduled 1765.

If the beat is determined 1750 to be a captured beat, then the cardiac signal represents a captured response signal and is used to update 1755 the capture detection interval and/or one or more variability thresholds respectively associated with features of the captured response. For example, the captured response peak timing may be used to update the range and/or midpoint of the classification interval. The peak timing of the capture response signal may be used to update the captured response peak timing variability threshold. The peak amplitude of the captured response signal may be used to update the captured amplitude variability threshold.

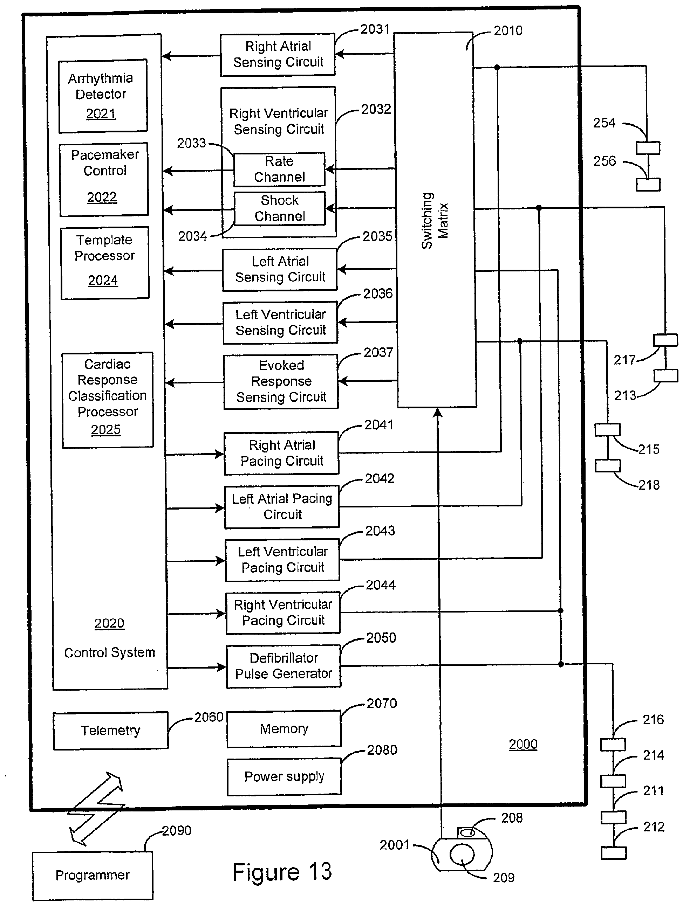

Referring now to Figure 13, there is shown an embodiment of a cardiac pacemaker/defibrillator 2000 suitable for implementing an atrial capture verification and retrograde management methodologies of the present invention. Figure 13 shows a cardiac pacemaker/defibrillator divided into functional blocks. It is understood by those skilled in the art that there exist many possible configurations in which these functional blocks can be arranged. The example depicted in Figure 13 is one possible functional arrangement. Other arrangements are also possible. For example, more, fewer or different functional blocks may be used to describe a cardiac pacemaker/defibrillator suitable for implementing the methodologies of the present invention. In addition, although the cardiac pacemaker/defibrillator 2000 depicted in Figure 13 contemplates the use of a programmable microprocessor-based logic circuit, other circuit implementations may be utilized.

The cardiac pacemaker/defibrillator 2000 depicted in Figure 13 includes circuitry for receiving cardiac signals from a heart and delivering electrical stimulation energy to the heart in the form of pacing pulses or defibrillation shocks. In one embodiment, the circuitry of the cardiac pacemaker/defibrillator 2000 is encased and hermetically sealed in a housing 2001 suitable for implanting in a human body. Power to the cardiac pacemaker/defibrillator 2000 is supplied by an electrochemical battery 2080. A connector block (not shown) is attached to the housing 1001 of the cardiac pacemaker/defibrillator 2000 to allow for the physical and electrical attachment of the lead system conductors to the circuitry of the cardiac pacemaker/defibrillator 2000.

The cardiac pacemaker/defibrillator 2000 may be a programmable microprocessor-based system, including a control system 2020 and a memory 2070. The memory 2070 may store parameters for various pacing, defibrillation, and sensing modes, along with other parameters. Further, the memory 2070 may store data indicative of cardiac signals received by other components of the cardiac pacemaker/defibrillator 2000. The memory 2070 may be used, for example, for storing historical EGM and therapy data. The historical data storage may include, for example, data obtained from long term patient monitoring used for trending or other diagnostic purposes. Historical data, as well as other information, may be transmitted to an external programmer unit 2090 as needed or desired.

The control system 2020 may cooperate with other components of the cardiac pacemaker/defibrillator 2000 to control the operations of the cardiac pacemaker/defibrillator 2000. In one example, the cardiac pacemaker/defibrillator 1000 may incorporate a sensor for determining the patient's hemodynamic need. The sensor output may be utilized by the control system 2020 to deliver pacing at a rate adapted to the activity level of the patient. In some implementations, the cardiac pacemaker/defibrillator 1000 may include components of an accelerometer and/or a transthoracic impedance sensor for determining the activity level and/or respiration rate of the patient. The control system 2020 depicted in Figure 13 incorporates a cardiac response classification processor 2025 for determining cardiac responses to pacing stimulation in accordance with various embodiments of the present invention. The control system 2020 may include additional functional components including a pacemaker control circuit 2022, an arrhythmia detector 2021, and a template processor 2024, along with other components for controlling the operations of the cardiac pacemaker/defibrillator 2000.

Telemetry circuitry 2060 may be implemented to provide communications between the cardiac pacemaker/defibrillator 2000 and an external programmer unit 2090. In one embodiment, the telemetry circuitry 1060 and the programmer unit 1090 communicate using a wire loop antenna and a radio frequency telemetric link, as is known in the art, to receive and transmit signals and data between the programmer unit 2090 and the telemetry circuitry 2060. In this manner, programming commands and other information may be transferred to the control system 2020 of the cardiac pacemaker/defibrillator 2000 from the programmer unit 2090 during and after implant. In addition, stored cardiac data pertaining to capture threshold, capture detection and/or cardiac response classification, for example, along with other data, may be transferred to the programmer unit 2090 from the cardiac pacemaker/defibrillator 2000.

In the embodiment of the cardiac pacemaker/defibrillator 1000 illustrated in Figure 13, electrodes RA-tip 256, RA-ring 254, RV-tip 212, RV-ring 211, RV-coil 214, SVC-coil 216, LV distal electrode 213, LV proximal electrode 217, LA distal electrode 218, LV proximal electrode 215, indifferent electrode 208, and can electrode 209, as illustrated in connection with Figure 2, are coupled through a switch matrix 2010 to sensing circuits 2031-2037.

A right atrial sensing circuit 2031 serves to detect and amplify electrical signals from the right atrium of the heart. Unipolar sensing may be implemented, for example, by sensing voltages developed between the RA-tip 256 and the can electrode 209. Outputs from the right atrial sensing circuit are coupled to the control system 2020.

A right ventricular sensing circuit 2032 serves to detect and amplify electrical signals from the right ventricle of the heart. Right ventricular cardiac signals sensed through use of the RV-tip 212 electrode are right ventricular near-field signals and are denoted RV rate channel signals. A bipolar RV rate channel signal may be sensed as a voltage developed between the RV-tip 212 and the RV-ring 211. Alternatively, bipolar sensing in the right ventricle may be implemented using the RV-tip electrode 212 and the RV-coil 214. Unipolar rate channel sensing in the right ventricle may be implemented, for example, by sensing voltages developed between the RV-tip 212 and the can electrode 209.

Right ventricular cardiac signals sensed through use of defibrillation electrodes are far-field signals, also referred to as RV morphology or RV shock channel signals. More particularly, a right ventricular shock channel signal may be detected as a voltage developed between the RV-coil 214 and the SVC-coil 216. A right ventricular shock channel signal may also be detected as a voltage developed between the RV-coil 214 and the can electrode 209. In another configuration the can electrode 209 and the SVC-coil electrode 216 may be electrically shorted and a RV shock channel signal may be detected as the voltage developed between the RV-coil 214 and the can electrode 209/SVC-coil 216 combination.

Left atrial cardiac signals may be sensed through the use of one or more left atrial electrodes 215, 918, which may be configured as epicardial electrodes. A left atrial ' sensing circuit 2035 serves to detect and amplify electrical signals from the left atrium of the heart. Bipolar sensing and/or pacing in the left atrium may be implemented, for example, using the LA distal electrode 218 and the LA proximal electrode 215. Unipolar sensing and/or pacing of the left atrium may be accomplished, for example, using the vector from the LA distal electrode 218 to can electrode 209 or the LA proximal electrode 215 to can electrode 209.

A left ventricular sensing circuit 2036 serves to detect and amplify electrical signals from the left ventricle of the heart. Bipolar sensing in the left ventricle may be implemented, for example, by sensing voltages developed between the LV distal electrode 213 and the LV proximal electrode 217. Unipolar sensing may be implemented, for example, by sensing voltages developed between the LV distal electrode 213 or the LV proximal electrode 217 to the can electrode 209.

Optionally, an LV coil electrode (not shown) may be inserted into the patient's cardiac vasculature, e.g., the coronary sinus, adjacent the left heart. Signals detected using combinations of the LV electrodes, 213, 217, LV coil electrode (not shown), and/or can electrodes 209 may be sensed and amplified by the left ventricular sensing circuitry 1036. The output of the left ventricular sensing circuit 2036 is coupled to the control system 1020.

The outputs of the switching matrix 2010 may be operated to couple selected combinations of electrodes 211, 212, 213, 214, 216, 217, 215, 918, 254, and 256 to an evoked response sensing circuit 2037. The evoked response sensing circuit 2037 serves to sense and amplify voltages developed using various combinations of electrodes for cardiac response classification in accordance with embodiments of the invention. In the embodiments described herein, various combinations of pacing and sensing electrodes may be utilized in connection with pacing and sensing the cardiac signal following the pace pulse to classify the cardiac response to the pacing pulse. For example, in some embodiments, a first electrode combination is used for pacing a heart chamber and a second electrode combination is used to sense the cardiac signal following pacing. In other embodiments, the same electrode combination is used for pacing and sensing. Further, the electrodes used to sense for the retrograde P-wave may be different or the same as the electrodes used to sense for the atrial evoked response.

The pacemaker control circuit 2022, in combination with pacing circuitry for the left atrium, right atrium, left ventricle, and right ventricle may be implemented to selectively generate and deliver pacing pulses to the heart using various electrode combinations. The pacing electrode combinations may be used to effect bipolar or unipolar pacing of the heart chambers as described herein.

Bipolar or unipolar pacing pulses may be delivered to a heart chamber using one of the pacing vectors as described herein. The electrical signal following the delivery of the pacing pulses may be sensed through various sensing vectors coupled through the switch matrix 2010 to the cardiac response classification processor 2025 and used to classify the cardiac response to pacing.

The switching matrix 2010 may be arranged to provide connections to various configurations of pacing and defibrillation electrodes. The outputs of the switching matrix 2010 may be coupled to an evoked response (ER) sensing circuit 2037 that serves to sense and amplify cardiac signals detected between the selected combinations of electrodes. The detected signals are coupled through the ER amplifier 2037 to a cardiac response classification processor 2025. The cardiac response classification processor 2025 includes circuitry configured to determine the cardiac response to a pacing stimulation. The presence or absence of an evoked response may be determined based on the amplitude, peak value, peak timing, and/or other morphological features of the cardiac signal sensed following the pacing pulse. The cardiac response classification methods described herein involve an adaptable classification interval that may be particularly useful in discriminating captured responses from fusion beats. Fusion management may be an important aspect in automatic capture verification. Further, erroneous classification of cardiac responses to pacing in capture threshold testing may produce either an underestimation or overestimation of the capture threshold. Erroneous classification of cardiac responses to pacing may result in the pacing energy being set too high or to low, resulting in an incorrect determination of the optimal pacing energy. The use of an adaptable classification interval rather than a fixed interval provides a classification interval that is adapted for each individual, thus providing more effective fusion management.

In accordance with various aspects of the invention, a cardiac signal following a pacing pulse is sensed. The sensed cardiac signal may be used to determine the cardiac response to the pacing pulse. Capture may be detected by comparing one or more characteristic features or samples of the cardiac signal following pacing to reference or template representative of a captured response. If the one or more signal features or samples are consistent with the template or reference value, capture is detected. For example, capture may be detected by comparing a peak value, e.g., a positive or negative peak value, of the signal sensed after delivery of the pacing pulse to a peak threshold representative of a captured response. If the absolute value of the signal peak meets or exceeds the captured peak threshold, capture is detected. Similarly, non-capture may be detected by comparing a positive or negative peak value of the signal sensed after delivery of the pacing pulse to a peak threshold representative of a non-captured response. If the absolute value of the signal peak remains below the non-captured peak threshold, non-capture is detected.

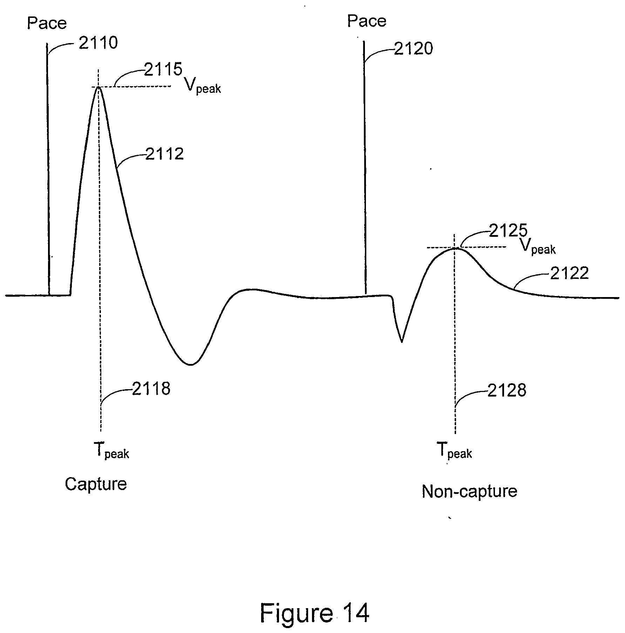

In some circumstances, multiple features or samples of the cardiac signal may be compared to a template for cardiac response determination. A cardiac signal may be considered to be consistent with a template if the features, samples, or morphological characteristics of the cardiac signal are determined to be sufficiently similar to the template features, samples, or morphological characteristics. If a cardiac signal is sufficiently similar to a template representative of a particular type of cardiac response, then the cardiac signal may be classified as that particular type of cardiac response. The graph of Figure 14 illustrates signals indicative of captured and non-captured responses to pacing. Pacing pulses 2110, 2120 may be delivered to an atrial or ventricular heart chamber. The cardiac signal 2112 sensed in the heart chamber following the first pacing pulse 2110 is a captured response, having a peak amplitude 2115 and peak timing 2118. The cardiac signal 2122 sensed following the second pacing pulse 2120 is a non-captured response having a peak amplitude 2125 and peak timing 2128. The captured response 2112 exhibits a relatively larger peak amplitude 2115 than the peak amplitude 2125 of the non-captured response. The relatively larger peak amplitude of the captured response signal can be used to discriminate between capture and non-capture. The timing 2118, 2128 of the peak amplitude of the captured or non-captured responses may be used along with the peak amplitude information to detect capture and/or non-capture.

Variability between patients, variability of device implantation configurations, and changes in patient conditions create significant issues for the creation and maintenance of templates and baseline references used for determining cardiac pacing responses. Further, templates or thresholds used for capture detection may be corrupted by the inclusion of noise, fusion beats or non-captured beats with intrinsic activity in a captured response template, for example. Embodiments of the present invention use median filtering methodologies to reduce spurious cardiac signals contributing to inaccuracies in captured response templates.

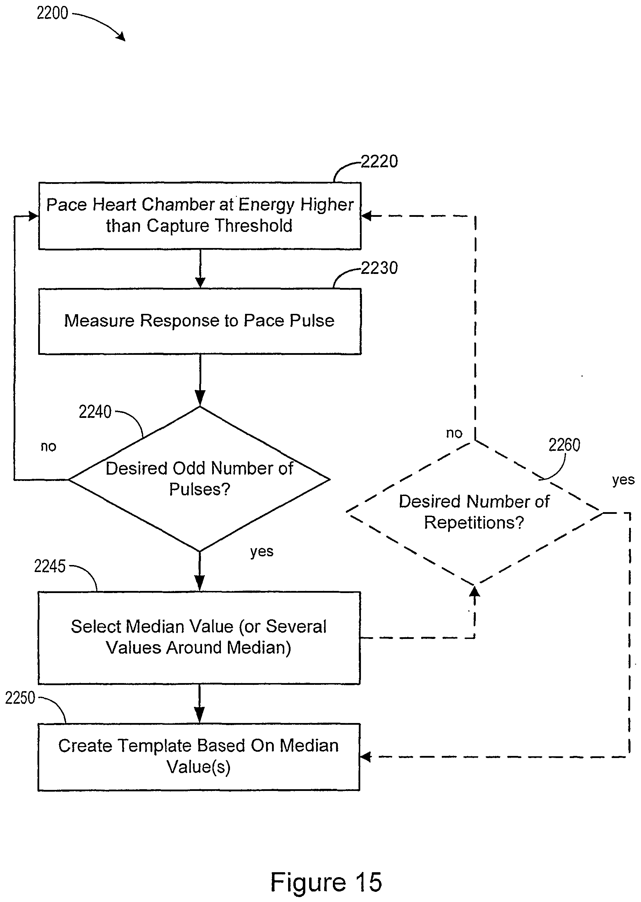

Figure 15 is a flow chart of a method 2200 of initializing a template in accordance with embodiments of the present invention. In an exemplary embodiment, a heart chamber is paced 2220 at a rate higher than the intrinsic rate, and at a level higher than the capture threshold. Jittering the pacing rate by varying the pacing rate slightly and/or randomly for each beat may be additionally or alternatively used for avoiding fusion. An odd number of pace pulses 2240 are used, and the evoked response 2230 is measured after each pace 2220. For example, the captured response peak amplitude and peak amplitude timing may be measured for each captured response 2230. The median value is then selected 2245 for use in template creation 2250. This process may be repeated 2260, and median values for each repetition 2260 may be averaged, to further reduce noise affecting the template. Alternately or additionally, several values around the median may be selected 2245 for a larger odd number of pace pulses, and used for averaging.

In order to increase the likelihood that fusion beats are avoided in the template creation 2250, an odd number, e.g., 33, is selected for the repetition 2240 count. Pacing 2220 and response measurement 2230 are repeated 33 times, and each time the values of the peak response amplitude and peak timing within a response interval are recorded by the CRM device. All the peak response amplitudes and peak timing values are then ranked in ascending or descending order respectively. The median ranked value, in this case the value having a ranking of 17, is then selected as the template value for an evoked response. In an alternate example, the median ranked value, having a ranking of 17, and the 4 values surrounding the median, in this case values having rankings 13-16 and 18-21, may be averaged to provide the evoked response template value.

In still a further embodiment, pacing 2220 and response measurement 2230 are repeated 9 times, and each time the values of the peak response amplitude and peak timing within a response interval are recorded by the CRM device. All the peak response amplitudes and peak timing values are then ranked in ascending or descending order respectively. The median ranked value, in this case the value having a ranking of 5, is then selected as the first candidate template value for an evoked response template statistical analysis. The pacing 2220 and response measurement 2230 are repeated 9 more times, values ranked, and the value having a ranking of 5 is selected again, this time as the second candidate template value. This process may be repeated 2260 for any desired repetitions of captured response analysis, until it is determined that the captured response candidate value(s) are representative of a true evoked response. The determination may be made after averaging a sufficient number of candidate values, after convergence is determined, or using other appropriate statistical principals.

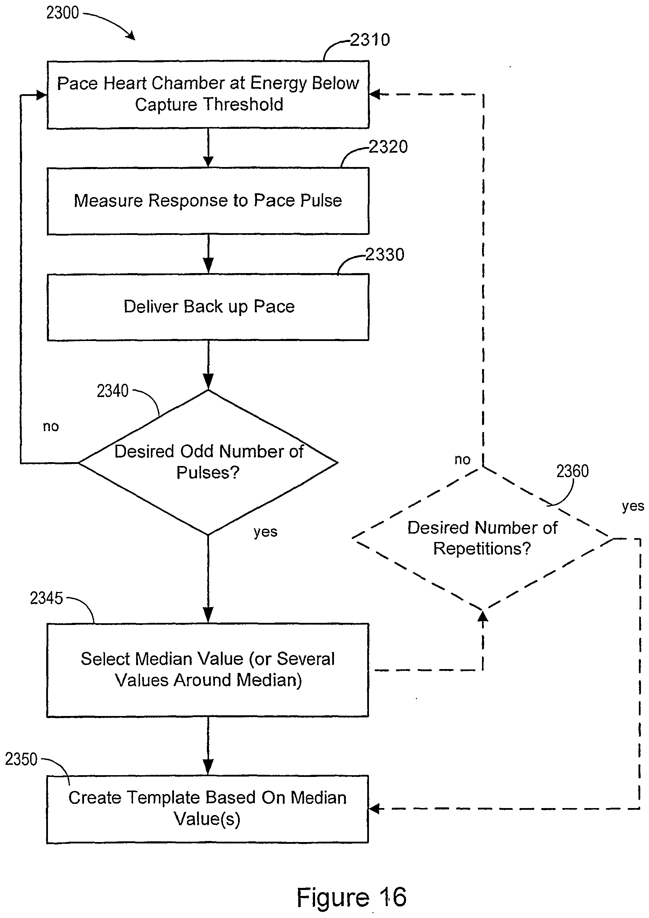

Figure 15 is a flow chart of a method 2200 of initializing a captured response template in accordance with embodiments of the present invention. Other types of cardiac response templates may also be created, updated, and/or refined using median filtering methodologies in accordance with the present invention. For example, as illustrated in Figure 16, a non-captured response template may be formed using the methodologies of the present invention. As illustrated in Figure 16, the heart may be paced 2310 at a rate higher than the intrinsic rate, but at an energy level lower than the capture threshold. Jittering the pacing rate by varying the pacing rate slightly and/or randomly for each beat may be additionally or alternatively used for avoiding fusion.

Features of the cardiac signal following and associated with the pacing pulse are measured 2320. For example, the peak amplitude and/or peak timing of the signal following the low level paces may be measured 2320. Because pacing pulses delivered below the capture threshold will not produce capture, a back up pace may be delivered 2330 after each primary pace 2310 to ensure continuous pacing therapy delivery to the patient. The back up pace may be delivered 2330 about 80 ms to about 100 ms following delivery of the primary pacing pulse.

An odd number of pacing pulses are delivered 2340 and the system determines median values 2345 of the measured amplitude, peak timing, or other measured feature value of the cardiac signals following the low level paces 2310. The median values of the peak amplitude and/or peak timing measurements are selected 2345 to create 2350 a non-captured response template. The number of low level paces used to form the template is preferably an odd number 2340. The larger the odd number 2340 of paces, the greater the chance of avoiding fusion beats.

The process of delivering a pacing pulse, measuring feature values and determining a median value of the measured feature value may be repeated 2360, and the median values for each repetition 2360 may be averaged, to further reduce noise affecting the template. Alternately or additionally, several values around the median may be selected 2345, and used for averaging.

Combinations of the methodologies exemplified in Figures 15-16 may also be used in accordance with the present invention. For example, a cardiac response to pacing may involve the utilization of one or both a non-captured response template and a captured response template to determine the cardiac pacing response. Median template creation methodologies in accordance with the present invention may also be useful for fusion detection and/or management. For example, if a cardiac signal sensed following pacing is inconsistent with a non-captured response template and a captured response template, the cardiac pacing response may be classified as a fusion or and unknown (possibly noise) response triggering a fusion/noise management process. Median filtering methodologies for cardiac response template generation in accordance with the present invention may also utilize adaptive adapt baselines for threshold measurements as described in more detail herein.

For purposes herein, the use of an odd number of pace pulses to find a median value shall be equivalent to providing an even number of pulses and disregarding or eliminating one or more pulses to arrive at an odd number, in order to define a median. Further, determining a median value shall be equivalent to ranking or ordering an odd or even number of responses and disregarding or eliminating a number of responses at the top and/or bottom of the rankings, effectively arriving at the approximation of a median or grouping around a median in accordance with the present invention.

Median template creation methodologies in accordance with the present invention may be used for both atrial and ventricular capture verification. Embodiments of the present invention may be useful with pacing pulses delivered to any heart chamber. For example, the pacing stimulation may be delivered to one or more of the right ventricle, the left ventricle, the right atrium, and the left atrium. Various embodiments of the invention involve using the same electrode combination for pacing and sensing. Other embodiments involve using an electrode combination for pacing that is different from the electrode combination used to sense the cardiac signal following pacing.

Variability between patients, variability of device implantation configurations, and changes in patient conditions create significant issues for both the creation and maintenance of templates and for capture verification. In order to detect captured response reliably, wide-band amplifiers may be used for cardiac response detection circuits. Baselines for such wide-band amplifiers often fluctuate beat-to-beat, especially when the pacing rate is high. Fluctuation at high pacing rates may be at least partly due to larger T-waves that occur relatively later than with lower pacing rates. The wide-band amplifier retains some of the T-wave spectrum, contributing the fluctuation. This fluctuation may lead to false detection of capture or loss of capture.

Fluctuation of in the cardiac signal baseline may lead to errors in peak amplitude measurement. Fluctuation of the baseline may be problematic in atrial and/or ventricular evoked response detection when pacing rate is high. Fluctuation of baselines is also problematic in some evoked response sensing configuration, for example, when a ventricle is paced and an A-ring to V-tip electrode configuration is used for sensing. Using an adaptive baseline in accordance with embodiments of the present invention provides improved captured response detection. An adaptive baseline may be used beat-by-beat, to reduce errors in captured response detection due to these fluctuations. Further, an adaptive baseline may be combined with the median filtering approach for template initialization described herein.

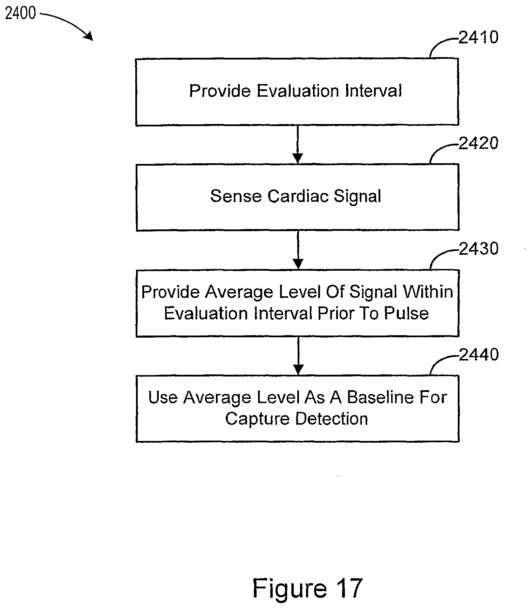

Figure 17 is a flowchart of a method 2400 of adapting a baseline. The method 2400 measures 2420 the cardiac signal during a moving evaluation interval 2410 prior to the pacing pulse. The cardiac signal measured 2420 during the moving interval is averaged 2430 and the average value is used as a baseline for the cardiac signal sensed following the pacing pulse. For example, a 3 to 5 millisecond interval (and possibly up to 10 milliseconds or more) may be used as a moving interval. The cardiac signal may, for example, be continuously evaluated for the average signal level within the moving evaluation interval. The average signal level 2430 may be determined by, for example, numerical averaging of digitized signals, using a low-pass filter and voltage hold circuit, or other filtering methodologies. The adaptive baseline serves as a reference point for measuring the cardiac signal peak sensed following delivery of the pacing pulse and used for detection of capture.

The adaptive baseline is particularly useful if a wideband amplifier is used for sensing cardiac signals used for cardiac response classification. In this scenario, the variation of the reference point for evoked response detection may vary significantly beat to beat, making an adaptive baseline for each beat desired. For example, in atrial pacing, for example, using the A-ring to V-tip sensing vector, the atrial pace can start before the signal (T-wave) returns to the nominal value. The residual signal from the T-wave may affect the evoked response detection. The adaptive baseline allows for higher pacing rates, for example, in atrial pacing.

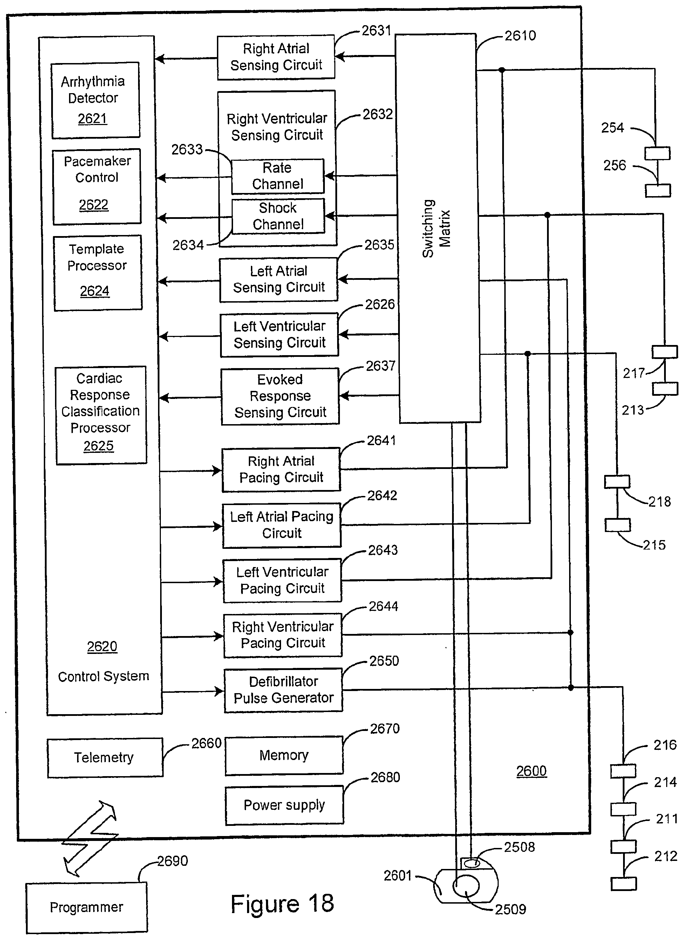

Referring now to Figure 18, there is shown an embodiment of a cardiac pacemaker/defibrillator 2600 suitable for implementing adaptable baselines for evoked response detection and initialization of templates for evoked response detection. Figure 18 shows a cardiac rhythm management device exemplified as a pacemaker/defibrillator divided into functional blocks. It is understood by those skilled in the art that there exist many possible configurations in which these functional blocks can be arranged. The example depicted in Figure 18 is one possible functional arrangement. Other arrangements are also possible. In addition, although the cardiac pacemaker/defibrillator 2600 depicted in Figure 18 contemplates the use of a programmable microprocessor-based logic circuit, other circuit implementations may be utilized.
The cardiac pacemaker/defibrillator 2600 depicted in Figure 18 includes circuitry for receiving cardiac signals from a heart and delivering electrical stimulation energy to the heart in the form of pacing pulses or defibrillation shocks. In one embodiment, the circuitry of the cardiac pacemaker/defibrillator 1800 is encased and hermetically sealed in a housing 2601 suitable for implanting in a human body. Power to the cardiac pacemaker/defibrillator 2600 is supplied by an electrochemical battery 2680. A connector block (not shown) is attached to the housing 2601 of the cardiac pacemaker/defibrillator 2600 to allow for the physical and electrical attachment of the lead system conductors to the circuitry of the cardiac pacemaker/defibrillator 2600.
The cardiac pacemaker/defibrillator 2600 may be a programmable microprocessor-based system, including a control system 2620 and a memory 2670. The memory 2670 may store parameters for various pacing, defibrillation, and sensing modes, along with other parameters. Further, the memory 2670 may store data indicative of cardiac signals received by other components of the cardiac pacemaker/defibrillator 2600. The memory 2670 may be used, for example, for storing historical EGM and therapy data. The historical data storage may include, for example, data obtained from long term patient monitoring used for trending or other diagnostic purposes. Historical data, as well as other information, may be transmitted to an external programmer unit 2690 as needed or desired.

The control system 2620 and memory 2670 may cooperate with other components of the cardiac pacemaker/defibrillator 2600 to control the operations of the cardiac pacemaker/defibrillator 2600. The control system depicted in Figure 18 incorporates a cardiac response classification processor 2625 for classifying cardiac responses to pacing stimulation in accordance with various embodiments of the present invention. The control system 2620 may include additional functional components including a pacemaker control circuit 2622, an arrhythmia detector2 621, and a template processor 2624, along with other components for controlling the operations of the cardiac pacemaker/defibrillator 2600. Telemetry circuitry 2660 may be implemented to provide communications between the cardiac pacemaker/defibrillator 2600 and an external programmer unit 2690. In one embodiment, the telemetry circuitry 2660 and the programmer unit 2690 communicate using a wire loop antenna and a radio frequency telemetric link, as is known in the art, to receive and transmit signals and data between the programmer unit 2690 and the telemetry circuitry 2660. In this manner, programming commands and other information may be transferred to the control system 2620 of the cardiac pacemaker/defibrillator 2600 from the programmer unit 2690 during and after implant. In addition, stored cardiac data pertaining to capture threshold, capture detection and/or cardiac response classification, for example, along with other data, may be transferred to the programmer unit 2690 from the cardiac pacemaker/defibrillator 2600.

In the embodiment of the cardiac pacemaker/defibrillator 2600 illustrated in Figure 18, electrodes RA-tip 256, RA-ring 254, RV-tip 212, RV-ring 211, RV-coil 214, SVC-coil 216, LV distal electrode 213, LV proximal electrode 217, LA distal electrode 218, Lv proximal electrode 215, indifferent electrode 208, and can electrode 209, as previously depicted in connection with Figure 2, are coupled through a switch matrix 2610 to sensing circuits 2631- 2637.

A right atrial sensing circuit 2631 serves to detect and amplify electrical signals from the right atrium of the heart. Unipolar sensing may be implemented, for example, by sensing voltages developed between the RA-tip 256 and the can electrode 209. Outputs from the right atrial sensing circuit are coupled to the control system 2620.

A right ventricular sensing circuit 2632 serves to detect and amplify electrical signals from the right ventricle of the heart. The right ventricular sensing circuit 2632 may include, for example, a right ventricular rate channel 2633 and a right ventricular shock channel 2634. Right ventricular cardiac signals sensed through use of the RV-tip 212 electrode are right ventricular near-field signals and are denoted RV rate channel signals. A bipolar RV rate channel signal may be sensed as a voltage developed between the RV-tip 212 and the RV-ring 211. Alternatively, bipolar sensing in the right ventricle may be implemented using the RV-tip electrode 212 and the RV-coil 214. Unipolar rate channel sensing in the right ventricle may be implemented, for example, by sensing voltages developed between the RV-tip 212 and the can electrode 209.

Right ventricular cardiac signals sensed through use of the RV-coil electrode 214 are far-field signals, also referred to as RV morphology or RV shock channel signals. More particularly, a right ventricular shock channel signal may be detected as a voltage developed between the RV-coil 214 and the SVC-coil 216. A right ventricular shock channel signal may also be detected as a voltage developed between the RV-coil 214 and the can electrode 209. In another configuration the can electrode 509 and the SVC-coil electrode 216 may be electrically shorted and a RV shock channel signal may be detected as the voltage developed between the RV-coil 214 and the can electrode 209/SVC-coil 216 combination.

Outputs from the right ventricular sensing circuit 2632 are coupled to the control system 2620. In one embodiment of the invention, rate channel signals and shock channel signals may be used to develop morphology templates for analyzing cardiac signals. In this embodiment, rate channel signals and shock channel signals may be transferred from the right ventricular sensing circuit 2632 to the control system 2620 and to a template processor 2624 where the morphological characteristics of a cardiac signal are analyzed. The template processor 2624 works in combination with the control system 2620 and the memory 2670 to generate and maintain various types of templates, including, for example, templates used for arrhythmia discrimination as well as cardiac response classification as described in more detail herein.

Left atrial cardiac signals may be sensed through the use of one or more left atrial electrodes 215, 218, which may be configured as epicardial electrodes. A left atrial sensing circuit 2635 serves to detect and amplify electrical signals from the left atrium of the heart. Bipolar sensing and/or pacing in the left atrium may be implemented, for example, using the LA distal electrode 218 and the LA proximal electrode 215. Unipolar sensing and/or pacing of the left atrium may be accomplished, for example, using the vector from the LA distal electrode 218 to can electrode 209 or the LA proximal electrode 215 to can electrode 209.

A left ventricular sensing circuit 2636 serves to detect and amplify electrical signals from the left ventricle of the heart. Bipolar sensing in the left ventricle may be implemented, for example, by sensing voltages developed between the LV distal electrode 213 and the LV proximal electrode 217. Unipolar sensing may be implemented, for example, by sensing voltages developed between the LV distal electrode 213 or the LV proximal electrode 217 to the can electrode 209.

Optionally, an LV coil electrode (not shown) may be inserted into the patient's cardiac vasculature, e.g., the coronary sinus, adjacent the left heart. Signals detected using combinations of the LV electrodes, 213, 217, LV coil electrode (not shown), and/or can electrodes 209 may be sensed and amplified by the left ventricular sensing circuitry 2636. The output of the left ventricular sensing circuit 2636 is coupled to the control system 2620.

The outputs of the switching matrix 610 may be operated to couple selected combinations of electrodes 211, 212, 213, 214, 216, 217, 215, 218, 254, and 256 to an evoked response sensing circuit 2637. The evoked response sensing circuit 2637 serves to sense and amplify voltage developed using various combinations of electrodes for cardiac response classification in accordance with embodiments of the invention.

In embodiments described herein, various combinations of pacing and sensing electrodes may be utilized in connection with pacing and sensing the cardiac signal following the pace pulse to classify the cardiac response to the pacing pulse. For example, in some embodiments, a first electrode combination is used for pacing a heart chamber and a second electrode combination is used to sense the cardiac signal following pacing. In other embodiments, the same electrode combination is used for pacing and sensing.

Sensing the cardiac signal following a pacing pulse using the same electrode combination for both pacing and sensing may yield a sensed cardiac signal including a pacing artifact component associated with residual post pace polarization at the electrode-tissue interface. The pacing artifact component may be superimposed on a smaller signal indicative of the cardiac response to the pacing pulse, i.e., the evoked response. The pacing output circuitry may include a coupling capacitor to block DC components from the heart and to condition the pacing stimulus pulse. A relatively large coupling capacitor may cause a larger pacing artifact that decays exponentially over a relatively long period of time.

The pacemaker control circuit 2622, in combination with pacing circuitry for the left atrium, right atrium, left ventricle, and right ventricle may be implemented to selectively generate and deliver pacing pulses to the heart using various electrode combinations. The pacing electrode combinations may be used to effect bipolar or unipolar pacing of the heart chambers as described herein.

Bipolar or unipolar pacing pulses may be delivered to a heart chamber using one of the pacing vectors as described herein. The electrical signal following the delivery of the pacing pulses may be sensed through various sensing vectors coupled through the switch matrix 2610 to the evoked response sensing circuit 2637 and used to classify the cardiac response to pacing.

In one example, the cardiac signal following the pacing pulse may be sensed using the same vector as was used for delivery of the pacing pulse. An adaptable cardiac response classification interval may be defined following the pacing pulse and used to classify the cardiac response to pacing. The adaptable cardiac response classification interval may be used to classify the cardiac response to pacing as one of a captured response, a non-captured response, and a fusion beat, for example.

Subcutaneous electrodes may provide additional sensing vectors useable for cardiac response classification. In one implementation, cardiac rhythm management system may involve a hybrid system including a first device, e.g. a pacemaker coupled to an intracardiac lead system, configured to pace the heart, and a second device, e.g. a pacemaker/defibrillator coupled to a subcutaneous lead system, configured to perform functions other than pacing. The second device may be employed to detect and classify cardiac responses to pacing based on signals sensed using subcutaneous electrode arrays. The first and second devices may operate cooperatively with communication between the devices occurring over a wireless link, for example. Examples of subcutaneous electrode systems and devices are described in commonly owned U.S. patent applications 10/462,001, filed June 13,2003 and 10/465,520, filed June 19, 2003. For right ventricular pacing, bipolar pacing may be delivered using the RV-tip electrode 212 and the RV-ring electrode 211. Unipolar pacing may be delivered using the RV-tip 212 to can electrode 209 vector. Sensing electrode combinations for cardiac response classification following RV pacing include RV-coil 214 to SVC-coil 216 tied to the can electrode 209, RV-coil 214 to can electrode 209, and, if the system includes a left ventricular lead, LV distal electrode 213 to LV proximal electrode 217.

In an example of left ventricular pacing, bipolar pacing pulses may be delivered to the left ventricle between the LV distal electrode 213 and the LV proximal electrode 217. In another example, unipolar pacing pulses may be delivered to the left ventricle, for example, between the LV distal electrode 213 and the can electrode 209. The cardiac signal following the delivery of the pacing pulses may be sensed using the LV proximal electrode 217 and the can electrode 209.

In an example of right atrial pacing, bipolar pacing pulses may be delivered to the right atrium between the RA-tip electrode 256 and the RA-ring electrode 254. In another example, unipolar pacing pulses may be delivered to the right atrium, for example, between the RA-tip electrode 256 and the can electrode 209. For unipolar right atrial pacing, an electrode combination useful for sensing cardiac signals following pacing for cardiac response classification includes the RA-ring 254 to indifferent electrode. In an example of left atrial pacing, bipolar pacing pulses may be delivered to the left atrium between the LA distal electrode 218 and an LA proximal electrode 215 In another example, unipolar pacing pulses may be delivered to the left atrium, for example, between the LA distal electrode 218 and the can electrode 209. The cardiac signal following the delivery of the pacing pulses and used for atrial capture verification may be sensed using the RA-tip 256 to RA-ring 254 vector.

In one embodiment a switching matrix 2610 is coupled to the RA-tip 256, RA-ring 254, RV-tip 212, RV-coil 214, LV distal electrode 213, LV proximal electrode 217, SVC coil 216, LA distal electrode 218, LA proximal electrode 215, indifferent 208, and can electrode 209. The switching matrix 2610 may be arranged to provide connections to various configurations of pacing and defibrillation electrodes. The outputs of the switching matrix 2610 are coupled to an evoked response (ER) sensing circuit 2637 that serves to sense and amplify cardiac signals detected between the selected combinations of electrodes. The detected signals are coupled through the ER amplifier 2637 to a cardiac response classification processor 2625. The cardiac response classification processor 2625 includes circuitry configured to adapt a cardiac response classification interval and to classify the cardiac response to a pacing stimulation, including, for example, classifying captured responses, non-captured responses, and fusion responses, in accordance with embodiments of the invention.

A number of the examples presented herein involve block diagrams illustrating functional blocks used for coordinated monitoring, diagnosis and/or therapy functions in accordance with embodiments of the present invention. It will be understood by those skilled in the art that there exist many possible configurations in which these functional blocks can be arranged and implemented. The examples depicted herein provide examples of possible functional arrangements used to implement the approaches of the invention.

It is understood that the components and functionality depicted in the figures and described herein can be implemented in hardware, software, or a combination of hardware and software. It is further understood that the components and functionality depicted as separate or discrete blocks/elements in the figures in general can be implemented in combination with other components and functionality, and that the depiction of such components and functionality in individual or integral form is for purposes of clarity of explanation, and not of limitation.

Various modifications and additions can be made to the preferred embodiments discussed hereinabove without departing from the scope of the present invention. Accordingly, the scope of the present invention should not be limited by the particular embodiments described above, but should be defined only by the claims set forth below.

## Claims

1. A medical system for classifying a cardiac response to a pacing pulse, comprising:
- a pacing circuit (2041-2044) configured to deliver (2220, 2310) pacing pulses to a heart chamber;
- a sensing circuit (2031-2037) configured to sense (2230, 2320) cardiac signals associated with the pacing pulses;
- a template processor (2024) configured to generate (2250, 2350) at least one cardiac response template by repetitively measuring (2230, 2320) cardiac signal feature values for each of a plurality of cardiac signals respectively associated with a plurality of pacing pulses and to determine (2245, 2345) a median value of the cardiac signal feature values for each repetition and to create (2250, 2350) the at least one cardiac response template by averaging the median values from each repetition; and
- a cardiac response classification processor (2025) configured to determine whether a sensed cardiac signal following a pacing pulse is consistent with the at least one cardiac response template and to classify the cardiac response to the pacing pulse based on whether the cardiac signal is consistent with the at least one cardiac response template.

2. A medical system for classifying a cardiac response to a pacing pulse, comprising:
- a pacing circuit (2041-2044) configured to deliver (2220, 2310) pacing pulses to a heart chamber;
- a sensing circuit (2031-2037) configured to sense (2230, 2320) cardiac signals associated with the pacing pulses;
- a template processor (2024) configured to generate (2250, 2350) at least one cardiac response template by repetitively measuring (2230, 2320) cardiac signal feature values for each of a plurality of cardiac signals respectively associated with a plurality of pacing pulses and to determine (2245, 2345) a median value of the cardiac signal feature values for each repetition and to create (2250, 2350) the at least one cardiac response template by averaging several values around each median value from each repetition; and
- a cardiac response classification processor (2025) configured to determine whether a sensed cardiac signal following a pacing pulse is consistent with the at least one cardiac response template and to classify the cardiac response to the pacing pulse based on whether the cardiac signal is consistent with the at least one cardiac response template.

3. The medical system of claims 1 or 2, wherein the at least one cardiac response template comprises a non captured response template.

4. The medical system of claim 3, wherein the at least one cardiac response template comprises a captured response template.

5. The medical system of claim 4, wherein the cardiac response classification processor (2025) is configured to classify the cardiac signal as fusion if the cardiac signal is not consistent with both the non captured response template and the captured response template.

6. The medical system of claims 1 or 2, wherein a pacing rate of the plurality of pacing pulses is jittered.

7. The medical system of claims 1 or 2, wherein the template processor is configured to determine the median value for each repetition by ranking the cardiac signal feature values and eliminating one or more of the cardiac signal values at the top and/or bottom of the ranking before determining the median value.

8. The medical system of claims 1 or 2, wherein the cardiac signal feature values comprise peak timing values.

9. The medical system of claims 1 or 2, wherein the cardiac signal feature values comprise peak amplitude values.

## Patentansprüche

1. Medizinisches System zum Klassifizieren einer Herzantwort auf einen Schrittgabeimpuls, welches aufweist:
- eine Schrittgabeschaltung (2041- 2044), die konfiguriert ist zum Liefern (2220, 2310) von Schrittgabeimpulsen zu einer Herzkammer;
- eine Erfassungsschaltung (2031- 2037), die konfiguriert ist zum Erfassen (220, 2320) von Herzsignalen, die mit den Schrittgabeimpulsen assoziiert sind;
- einen Schablonenprozessor (2024), der konfiguriert ist zum Erzeugen (2250, 2350) von zumindest einer Herzantwortschablone durch wiederholtes Messen (2230, 2320) von Herzsignal-Merkmalswerten für jedes von mehreren Herzsignalen, die jeweils mit mehreren Schrittgabeimpulsen assoziiert sind, und zum Bestimmen (2245, 2345) eines Medianwerts der Herzsignal-Merkmalswerte für jede Wiederholung und zum Schaffen (2250, 2350) der zumindest einen Herzantwortschablone durch Bilden des Durchschnitts der Medianwerte von jeder Wiederholung; und
- einen Herzantwort-Klassifizierungsprozessor (2025), der konfiguriert ist zum Bestimmen, ob ein erfasstes Herzsignal folgend einem Schrittgabeimpuls mit der zumindest einen Herzantwortschablone übereinstimmend ist, und zum Klassifizieren der Herzantwort auf den Schrittgabeimpuls auf der Grundlage dessen, ob das Herzsignal mit der zumindest einen Herzantwortschablone übereinstimmt.

2. Medizinisches System zum Klassifizieren einer Herzantwort auf einen Schrittgabeimpuls, welches aufweist:
- eine Schrittgabeschaltung (2041- 2044), die konfiguriert ist zum Liefern (2220, 2310) von Schrittgabeimpulsen zu einer Herzkammer;
- eine Erfassungsschaltung (2041- 2037), die konfiguriert ist zum Erfassen (2230, 2320) von mit den Schrittgabeimpulsen assoziierten Herzsignalen;
- einen Schablonenprozessor (2024), der konfiguriert ist zum Erzeugen (2250, 2350) von zumindest einer Herzantwortschablone durch wiederholtes Messen (2230, 2320) von Herzsignal-Merkmalswerten für jedes von mehreren Herzsignalen, die jeweils mit mehreren Schrittgabeimpulsen assoziiert sind, und zum Bestimmen (2245, 2345) eines Medianwerts der Herzsignal-Merkmalswerte für jede Wiederholung und zum Schaffen (2250, 2350) der zumindest einen Herzantwortschablone durch Bilden des Durchschnitts mehrerer Werte um jeden Medianwert von jeder Wiederholung herum; und
- einen Herzantwort-Klassifizierungsprozessor (2025), der konfiguriert ist zum Bestimmen, ob ein erfasstes Herzsignal folgend einem Schrittgabeimpuls mit der zumindest einen Herzantwortschablone übereinstimmt, und zum Klassifizieren der Herzantwort auf den Schrittgabeimpuls auf der Grundlage dessen, ob das Herzsignal mit der zumindest einen Herzantwortschablone übereinstimmt.

3. Medizinisches System nach den Ansprüchen 1 oder 2, bei dem die zumindest eine Herzantwortschablone eine nicht erfasste Antwortschablone aufweist.

4. Medizinisches System nach Anspruch 3, bei dem die zumindest eine Herzantwortschablone eine erfasste Antwortschablone aufweist.

5. Medizinisches System nach Anspruch 4, bei dem der Herzantwort-Klassifizierungsprozessor (2025) konfiguriert ist zum Klassifizieren des Herzsignals als Fusion, wenn das Herzsignal sowohl mit der nicht erfassten Antwortschablone als auch der erfassten Antwortschablone nicht übereinstimmt.

6. Medizinisches System nach Anspruch 1 oder 2, bei dem eine Schrittgaberate der mehreren Schrittgabeimpulse zittert.

7. Medizinisches System nach Anspruch 1 oder 2, bei dem der Schablonenprozessor konfiguriert ist zum Bestimmen des Medianwerts für jede Wiederholung durch Bilden einer Rangordnung der Herzsignal-Merkmalswerte und Eliminieren eines oder mehrerer der Herzsignalwerte am oberen und/oder unteren Ende der Rangordnung, bevor der Medianwert bestimmt wird.

8. Medizinisches System nach Anspruch 1 oder 2, bei dem die Herzsignal-Merkmalswerte Spitzenzeitwerte aufweisen.

9. Medizinisches System nach Anspruch 1 oder 2, bei dem die Herzsignal-Merkmalswerte Spitzenamplitudenwerte aufweisen.

## Revendications

1. Système médical pour classifier une réponse cardiaque à une impulsion de stimulation cardiaque, comprenant :
- un circuit de stimulation cardiaque (2041 - 2044) qui est configuré de manière à délivrer (2220, 2310) des impulsions de stimulation cardiaque à une chambre de coeur ;
- un circuit de détection (2031 - 2037) qui est configuré de manière à détecter (2230, 2320) des signaux cardiaques qui sont associés aux impulsions de stimulation cardiaque ;
- un processeur de modèle (2024) qui est configuré de manière à générer (2250, 2350) au moins un modèle de réponse cardiaque en mesurant de façon répétée (2230, 2320) des valeurs de caractéristique de signal cardiaque pour chacun d'une pluralité de signaux cardiaques qui sont respectivement associés à une pluralité d'impulsions de stimulation cardiaque et de manière à déterminer (2245, 2345) une valeur médiane des valeurs de caractéristique de signal cardiaque pour chaque répétition et de manière à créer (2250, 2350) l'au moins un modèle de réponse cardiaque en calculant la moyenne des valeurs médianes provenant de chaque répétition ; et
- un processeur de classification de réponse cardiaque (2025) qui est configuré de manière à déterminer si oui ou non un signal cardiaque détecté qui suit une impulsion de stimulation cardiaque est cohérent avec l'au moins un modèle de réponse cardiaque et de manière à classifier la réponse cardiaque à l'impulsion de stimulation cardiaque sur la base de si oui ou non le signal cardiaque est cohérent avec l'au moins un modèle de réponse cardiaque.

2. Système médical pour classifier une réponse cardiaque à une impulsion de stimulation cardiaque, comprenant :
- un circuit de stimulation cardiaque (2041 - 2044) qui est configuré de manière à délivrer (2220, 2310) des impulsions de stimulation cardiaque à une chambre de coeur ;
- un circuit de détection (2031 - 2037) qui est configuré de manière à détecter (2230, 2320) des signaux cardiaques qui sont associés aux impulsions de stimulation cardiaque ;
- un processeur de modèle (2024) qui est configuré de manière à générer (2250, 2350) au moins un modèle de réponse cardiaque en mesurant de façon répétée (2230, 2320) des valeurs de caractéristique de signal cardiaque pour chacun d'une pluralité de signaux cardiaques qui sont respectivement associés à une pluralité d'impulsions de stimulation cardiaque et de manière à déterminer (2245, 2345) une valeur médiane des valeurs de caractéristique de signal cardiaque pour chaque répétition et de manière à créer (2250, 2350) l'au moins un modèle de réponse cardiaque en calculant la moyenne de plusieurs valeurs autour de chaque valeur médiane provenant de chaque répétition ; et
- un processeur de classification de réponse cardiaque (2025) qui est configuré de manière à déterminer si oui ou non un signal cardiaque détecté qui suit une impulsion de stimulation cardiaque est cohérent avec l'au moins un modèle de réponse cardiaque et de manière à classifier la réponse cardiaque à l'impulsion de stimulation cardiaque sur la base de si oui ou non le signal cardiaque est cohérent avec l'au moins un modèle de réponse cardiaque.

3. Système médical selon les revendications 1 ou 2, dans lequel l'au moins un modèle de réponse cardiaque comprend un modèle de réponse non captu rée.

4. Système médical selon la revendication 3, dans lequel l'au moins un modèle de réponse cardiaque comprend un modèle de réponse capturée.

5. Système médical selon la revendication 4, dans lequel le processeur de classification de réponse cardiaque (2025) est configuré de manière à classifier le signal cardiaque en tant que fusion si le signal cardiaque n'est pas cohérent avec à la fois le modèle de réponse non capturée et le modèle de réponse capturée.

6. Système médical selon les revendications 1 ou 2, dans lequel une fréquence de stimulation cardiaque de la pluralité d'impulsions de stimulation cardiaque présente une gigue/instabilité.

7. Système médical selon les revendications 1 ou 2, dans lequel le processeur de modèle est configuré de manière à déterminer la valeur médiane pour chaque répétition en classant les valeurs de caractéristique de signal cardiaque et en éliminant une ou plusieurs des valeurs de signal cardiaque au niveau du sommet et/ou du fond du classement avant de déterminer la valeur médiane.

8. Système médical selon les revendications 1 ou 2, dans lequel les valeurs de caractéristique de signal cardiaque comprennent des valeurs de cadencement/synchronisation de crête.

9. Système médical selon les revendications 1 ou 2, dans lequel les valeurs de caractéristique de signal cardiaque comprennent des valeurs d'amplitude de crête.
